# EUROPÄISCHE PATENTSCHRIFT

(11) **EP 0 837 056 B1**
(45) Veröffentlichungstag und Bekanntmachung des Hinweises auf die Patenterteilung: **08.01.2003**
(21) Anmeldenummer: 97117008.9
(22) Anmeldetag: 01.10.1997
(51) Int. Cl.: C07C 303/40, C07C 311/51, C07B 61/00

(54) **Kombinatorische Wirkstoffsynthese und deren Zwischenprodukte**
Combinatorial reagent synthesis and intermediates therefor
Synthèse combinatoire de réactif et intermédiaires

(30) Priorität: 15.10.1996 DE 19642429
(43) Veröffentlichungstag der Anmeldung: 22.04.1998
(73) Patentinhaber: Bayer CropScience GmbH, 65929 Frankfurt/Main (DE)
(72) Erfinder: Haaf, Klaus, Dr., 65779 Kelkheim (DE); Willms, Lothar, Dr., 65719 Hofheim (DE)

(56) Entgegenhaltungen:
- DE-B- 1 030 330
- US-A- 2 503 820
- US-A- 5 215 570
- H. ULRICH, ET AL.: "The reaction of sulphonylureas and sulphonamides with carbonyl chloride. A new synthesis of isocyanates" JOURNAL OF ORGANIC CHEMISTRY, Bd. 31, Nr. 8, August 1966 (1966-08), Seiten 2658-2661, XP002124609 American Chemical Society, Washington, DC, US ISSN: 0022-3263
- CHEMICAL ABSTRACTS, vol. 50, no. 1, 10. Januar 1956 (1956-01-10) Columbus, Ohio, US; abstract no. 364h, L. RAFFA: "Benzo-1,2,4-thiodiazine derivatives. I. Closing of the nucleus of 3-oxodihydrobenzo-1,2,4-thiodiazine 1,1-dioxide" Spalte 364; XP002124610 & II FARMACO, Bd. 9, 1954, Seiten 661-681, Rom, IT

## Beschreibung

Die Erfindung betrifft das technische Gebiet der Synthese von Wirkstoffen mit bestimmten gemeinsamen Strukturmerkmalen.

Die steigenden Anforderungen an die Eigenschaften neuer biologisch aktiver Stoffe für den Pflanzenschutz oder die Medizin haben es mit sich gebracht, daß die Entwicklung eines marktreifen Wirkstoffs mit der Herstellung und Prüfung immer größerer Zahlen an Testsubstanzen verbunden ist. Nach Einschätzung vieler Fachleute wird diese Tendenz voraussichtlich trotz verfeinerter Kenntnisse über die Biochemie bekannter Wirkstoffe und computerunterstützter Berechnungen von Molekülstrukturen und -eigenschaften ("molecular modelling") anhalten. Um den Kosten- und Zeitaufwand nicht gleichermaßen ansteigen zu lassen, stellt sich für die Forschung nach neuen Wirkstoffen die Aufgabe, effektivere Methoden zur Herstellung großer Zahlen neuartiger Testverbindungen zu entwickeln.

Die Methoden zur systematischen Herstellung großer Zahlen an Testverbindungen und speziell dazu geeigneter Methoden zu deren Analyse werden unter dem Begriff "kombinatorische Chemie" zusammengefaßt; vgl. z. B. J. S. Früchtel, G. Jung in Angew. Chem. 108 (1996) S. 19 ff.

Einige kombinatorische Synthesemethoden zielen darauf ab, in möglichst effektiver, standardisierter Weise eine große Zahl von strukturvarianten Verbindungen in möglichst wenigen Reaktionsschritten gemeinsam ("in einem Pool") herzustellen und gemeinsam auf biologische Wirkung zu testen; vgl. z. B. die Divide-Couple-and-Recombine-Methode nach a) K. S. Lam, S. E. Salmon, E. M. Hersh, V. J. Hruby, W. M. Kazmeiersky, R. J. Knapp in Nature 82 (1991) 354, b) A. Furka, F. Sebestyen, M. Asgedom, G. Dibo in Int. J. Pept. Protein Res. 37 (1991) 487. Falls dann eine ganze Gruppe von Verbindungen ("Pool") keine wirksame Verbindung enthält, so reicht ein einziger gemeinsamer Test aus, um diese Struktrurvarianten auszuschließen. Falls der gemeinsame Test jedoch Wirksamkeit anzeigt, kann die Variation bei der Herstellung der Testverbindungen gezielt verringert werden, um die Gruppe mit dem Wirkstoff oder den Wirkstoffen einzugrenzen und schließlich die Struktur der wirksamen Verbindungen zu bestimmen. Die beschriebene Methode läßt sich in der Regel jedoch nicht mehr sinnvoll anwenden, wenn es um die Optimierung von Wirkstoffstrukturen geht und in der Gruppe der Testverbindungen viele ähnlich wirksame Verbindungen vorhanden sind oder auch wenn größere Mengen der Verbindungen für die Ersttests benötigt werden.

Zur Lösung der letztgenannten Aufgabe geht man oftmals von einer Verbindung mit bekannter biologischer Wirkung aus, der sogenannten Leitstruktur oder Leitverbindung, und variiert die Struktur der Leitverbindung systematisch mit Hilfe eines weitgehend standardisierten Herstellungsverfahrens durch Einsatz einer großen Zahl unterschiedlicher Edukte. Die jeweils hergestellten Einzelverbindungen werden dann einzeln auf ihre biologische Wirkung getestet, um die Verbindung mit optimaler Wirkung gleicher Wirkungsart herauszufinden.

Zu den bekannten Synthesemethoden aus der kombinatorischen Chemie (siehe J. S. Früchtel, G. Jung in Angew. Chem. 108 (1996) S. 19 ff. und dort zitierter Literatur) gehört eine Gruppe von Methoden, bei welchen der jeweilige Wirkstoff schrittweise festkörpergebunden, insbesondere an einem synthetischen oder natürlichen Harz gebunden, hergestellt wird. Mit Hilfe der Bindung an den Festkörper, z. B. an das Harz in Form von Teilchen hoher Korngröße oder Kügelchen, werden die Zwischenstufen im Prinzip makroskopisch handhabbar. Die Synthese eines Wirkstoffes über mehrere Reaktionsstufen benötigt dann weniger Aufwand bei der Isolierung und Reinigung als bei konventionellen Methoden, weil diese Schritte in der Regel in Form einer einfachen Filtration und Abspülung der Harzkörper zu bewerkstelligen sind. Das harzgebundene fertige Wirkstoffmolekül muß schließlich noch vom Harz abgespalten werden.

Für die Wahl geeigneter Harz-Molekül-Systeme ergeben sich grundsätzlich Probleme durch den Zielkonflikt, sowohl eine gewünschte hohe Stabilität der Bindung zwischen synthetisierten Molekülteilen und Harz bei der Anwendung unterschiedlicher Reaktionstypen und -bedingungen zu gewährleisten als auch eine schonende Methode zur überwiegenden oder vollständigen Abspaltung des fertigen Synteseprodukts vom Harz zu ermöglichen.

Der Erfindung liegt die Aufgabe zugrunde, eine kombinatorische Synthesemethode auf Basis harzgebundener Synthesebausteine und -produkte bereitzustellen, welche die Synthese einer breiten Variation von biologisch aktiven Verbindungen mit gleicher Partialstruktur erlaubt.

Gegenstand der Erfindung ist ein Verfahren zur Herstellung von chemischen Verbindungen der Formel (I),

R¹-SO₂-NH-CO-R² (I)

worin R¹ und R² jeweils einen organischen Rest bedeuten,
dadurch gekennzeichnet, daß man
a) eine Harz-Linker-Verbindung der Formel (II),

   [Harzpolymer]-[L-Nuc]ₙ (II)

   worin
   - [Harzpolymer]: für den Rest eines Harzes steht, der über n Bindungsstellen mit den dargestellten n Gruppen der Formel -L-Nuc verbunden ist,
   - L: jeweils einen organischen Linker bedeutet,
   - Nuc: eine nucleofuge Gruppe (Abgangsgruppe) oder unter den Reaktionsbedingungen zu einer Abgangsgruppe zu aktivierenden Gruppe bedeutet, z. B. OH, Amino, Halogen, Mesyl oder Tosyl bedeutet,
   - n: die Anzahl der funktionellen Gruppen L-Nuc am Harz bedeutet, die vom Molekulargewicht des Harzpolymers abhängt und größer oder gleich 1 ist,
   mit einem Acylsulfonamid der Formel (III),

   E¹-SO₂-NH-CO-E² (III)

   worin E¹ und E² unabhängig voneinander jeweils einen für die Herstellung der Reste R¹ bzw. R² in Verbindung (I) geeigneten organischen Rest bedeuten,
   in Gegenwart eines Kondensationsmittels zu einem harzgebundenen Addukt der Formel (IV), 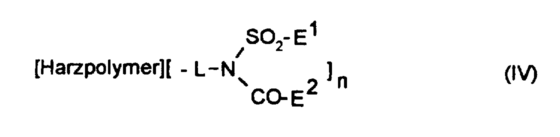 worin [Harzpolymer], L, n, E¹ und E² wie in Formel (II) bzw. Formel (III) definiert sind, umsetzt,
b) das erhaltene Addukt (IV) in einem oder mehreren weiteren Reaktionsschritten an den organischen Resten E¹ bzw. E² derivatisiert und damit gegebenenfalls über harzgebundene Zwischenprodukte der Formel (IV'), welche im Unterschied zu Formel (IV) die organischen Reste (E¹)' bzw. (E²)' der Derivate enthalten, zur Verbindung (IV"), 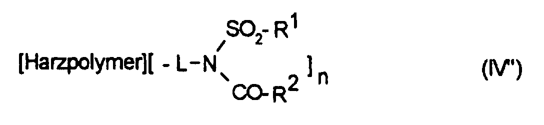 worin R¹ und R² wie in Formel (I) und [Harzpolymer], L und n wie in Formel (II) bzw. Formel (IV) definiert sind, umsetzt und
c) die Verbindung der Formel (I) aus dem Harz-Linker-Addukt der Formel (IV") abspaltet.

Gegenstand der Erfindung sind auch die Einzelschritte des erfindungsgemäßen Verfahrens und die neuartigen Addukte der Formel (IV) sowie der Formel (IV') und (IV''). Letztere beiden Verbindungsgruppen sind ebenfalls Verbindungen der allgemeinen Formel (IV), in denen jedoch die Reste E¹ und E² die Bedeutung der entsprechend modifizierten organischen Reste (E¹)' bzw. (E²)' und R¹ bzw. R² der derivatisierten Zwischenprodukte haben.

Ein Aspekt der Erfindung ist die in Schritt a) durchgeführte Anbindung von Verbindungen mit der Partialstruktur -SO₂-NH-CO- (Acylsulfonamid) an einen Harzkörper (Harzpolymer). Die Methode der Anbindung ermöglicht zugleich auch eine Methode zur weitgehenden oder vollständigen Abspaltung der Verbindungen unter Erhalt der Partialstruktur -SO₂-NH-CO- [siehe Schritt c)], wobei zwischen Anbindung und Abspaltung vom Harzkörper strukturelle Modifizierungen in anderen Molekülteilen vornommen werden können. Die besonderen Bedingungen werden durch die Funktion des Linkers erfüllt, dessen Struktur den Reaktionstyp für die chemische Anbindung und Abspaltung der Verbindungen mit der genannten Partialstruktur festlegt. Eine derartige Methode zur Anbindung von Verbindungen mit der Partialstruktur -SO₂-NH-CO- an einen Harzkörper war bisher nicht bekannt. Unter der Vielzahl der bekannten, prinzipiell möglichen Reaktionstypen für die Umsetzung eines Acylsulfonamids an dessen Amidogruppe, wie beispielsweise N-Alkylierung, N-Acylierung, Addition an Alkene und aktivierte Alkene etc., gibt es jedoch kaum eine geeignete Methode, die eine Abspaltung unter Erhalt der Acylsulfonamid-struktur ermöglicht. Viele der Reaktionen erfordern Bedingungen, welche mit dem Harzkörper nicht verträglich sind, z. B. zu hohe oder zu niedrige Temperaturen, stark basische oder saure Bedingungen. Der Harzkörper muß bei den Reaktionen quellfähig und in seiner Polymerstruktur weitgehend unverändert bleiben, weil er über mehrere Reaktionsschritte eingesetzt und nach der Reaktion oder Reaktionssequenz wieder abgetrennt werden muß.

Erfindungsgemäß lassen sich jedoch die Acylsulfonamide überraschenderweise mit einer Reihe von Linkern in geeigneter Weise an das Harzpolymer binden, deren Einsetzbarkeit aus den obengenannten Gründen nicht von vorherein anzunehmen war und die nunmehr weitere analoge Möglichkeiten eröffnen (Erläuterungen s. u.).

Gegenstand der Erfindung sind deshalb insbesondere die Reaktionstufen a) (Anbindung) und c) (Abspaltung) sowie die Harz-Linker-Addukte (IV), (IV') und (IV"). Die Derivatisierungsreaktion oder -reaktionen in der Stufe b) (Derivatisierung) sind in der Regel vom Typ her bekannt und können meist unter analogen Reaktionsbedingungen angewendet werden, wobei manche bevorzugte Verfahrensmaßnahmen durch Besonderheiten des Harzkörpers bedingt sind. Bei der Wahl der Derivatisierungsreaktionen sind mögliche Wechselwirkungen zwischen funktionellen Gruppen in den organischen Resten E¹ und E² bzw. (E¹)' und (E²)' bzw. R¹ und R² in der dem Fachmann bekannten Weise zu berücksichtigen. Eine generelle Einschränkung der Struktur der organischen Reste in den Verbindungen (I), (IV), (IV') und (IV'') besteht nicht.

In der Formel (I) und den anderen allgemeinen Formeln (III), (IV), (IV'), (IV'') usw. bedeutet ein organischer Rest R¹ oder R² einen kohlenstoffhaltigen Rest, beispielsweise einen gegebenenfalls substituierten (hetero)aromatischen Rest oder einen aliphatischen, d. h. nicht aromatischen organischen Rest, der außer C-Atomen und Wasserstoffatomen auch Heteroatome enthalten und/oder substituiert sein kann und der auch über Heteroatome mit anderen Molekülteilen verbunden sein kann, z. B. im Falle der Verbindungen der Formel (I) mit der SO₂-Gruppe oder der Carbonylgruppe über Heteroatome verbunden sein kann. Die geeigneten organischen Reste können in der Größe recht unterschiedlich sein; vorzugsweise enthält ein organischer Rest inklusive eventuell enthaltender Substituenten weniger als 30 C-Atome, insbesondere 1 bis 20 C-Atome, wobei kleinere Reste mit 1 bis 12 C-Atomen in der Regel bevorzugt sind. Als Substituenten eines organischen Restes kommen ebenfalls (hetero)aromatische und aliphatische Reste, inklusive funktionelle Gruppen in Frage, wobei die funktionellen Gruppen vorzugsweise gut mit den sonst in der Verbindung der Formeln (I) und (II) vorhandenen funktionellen Gruppen kompatibel sind. Beispielsweise sollten als funktionelle Gruppen keine oxidativen Gruppen vorhanden sein, wenn der Linker oxidationsempfindlich ist und damit schon unter den Bedingungen der kombinatorische Synthese reagieren würde.

Organische Reste sind beispielsweise gegebenenfalls substituierte Kohlenwasserstoffreste oder Kohlenwasserstoffoxyreste. Ein Kohlenwasserstoffrest ist ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl; vorzugsweise bedeutet ein Kohlenwasserstoffrest Alkyl, Alkenyl oder Alkinyl mit bis zu 12 C-Atomen oder Cycloalkyl mit 3, 4, 5, 6, 7 oder 8 Ringatomen oder Aryl; entsprechendes gilt für einen Kohlenwasserstoffrest in einem Kohlenwasserstoffoxyrest.
Aryl bedeutet ein mono-, bi- oder polycyclisches, carbocyclisches aromatisches Ringsystem; im substituierten Fall, oder genauer im cyclisch substituierten Fall, werden insbesondere auch bicyclische oder polycyclische Ringsysteme mit mindestes einem aromatischen Ring, der mit einem oder mehreren cycloaliphatischen, gegebenenfalls teilungesättigten Ringen anelliert ist, eingeschlossen. Gegebenfalls cyclisch substituiertes Aryl ist beispielsweise Phenyl, Naphthyl, Tetrahydronaphthyl, Indenyl, Indanyl, Pentalenyl, Fluorenyl und ähnliches, wobei die genannten Ringsysteme im allgemein substituierten Fall noch weiter substituiert sein können; vorzugsweise ist Aryl ein unsubstituierter Phenyloder Naphthylring; substituiertes Aryl ist vorzugsweise ein Phenylrest, der unsubstituiert oder substituiert ist, wobei die Substituenten keinen ankondensierten Ring bedeuten.

Heteroaryl oder ein heteroaromatischer Rest bedeutet ein mono-, bi- oder polycyclisches aromatisches Ringsystem, in dem mindestens 1 Ring ein oder mehrere Heteroatome enthält, beispielsweise Pyridyl, Pyrimidinyl, Pyridazinyl, Pyrazinyl, Triazinyl, Thienyl, Thiazolyl, Oxazolyl, Isoxazolyl, Furyl, Pyrrolyl, Pyrazolyl und Imidazolyl. Im substituierten Fall werden insbesondere auch bicyclische oder polycyclische aromatische, benzokondensierte oder mit cycloaliphatischen Ringen anellierte Verbindungen, z.B. Chinolinyl, Benzoxazolyl etc. eingeschlossen. Heteroaryl schließt auch einen heteroaromatischen Ring ein, der vorzugsweise 5- oder 6-gliedrig ist und 1, 2- oder 3 Heteroringatome, insbesondere aus der Gruppe N, O und S enthält.

Ein heterocyclischer Rest (Heterocyclyl) oder Ring (Heterocyclus) kann gesättigt, ungesättigt oder heteroaromatisch (Heteroaryl) sein; er enthält ein oder mehrere Heteroringatome, vorzugsweise aus der Gruppe N, O und S; vorzugsweise ist er ein nicht aromatischer Ring mit 3 bis 8 Ringatomen und 1 bis 3 Heteroringatomen aus der Gruppe N, O und S oder ist ein heteroaromatischer Ring mit 5 oder 6 Ringatomen und enthält 1, 2 oder 3 Heteroringatome aus der Gruppe N, O und S. Der Rest kann z.B. ein wie oben definierter heteroaromatischer Rest oder Ring sein oder ist ein partiell hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substitutiertes Alkyl, Alkenyl, Alkinyl, Aryl, Phenyl und Benzyl, oder substituiertes Heteroaryl, ein substituierter bicyclischer Rest oder Ring oder ein substituierter bicyclischer Rest, gegebenenfalls mit aromatischen Anteilen, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, substituiertes Amino wie Acylamino, Mono- oder Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, C₁-C₄-Alkyl, vorzugsweise Methyl oder Ethyl, C₁-C₄-Haloalkyl, vorzugsweise Trifluormethyl, C₁-C₄-Alkoxy, vorzugsweise Methoxy oder Ethoxy, C₁-C₄-Haloalkoxy, Nitro und Cyano. Besonders bevorzugt sind dabei die Substituenten Methyl, Methoxy und Chlor.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, C₁-C₄-Alkyl, C₁-C₄-Alkoxy, C₁-C₄-Halogenalkyl, C₁-C₄-Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Die Reste Alkyl, Alkoxy, Haloalkyl, Haloalkoxy, Alkylamino und Alkylthio sowie die entsprechenden ungesättigten und/oder substituierten Reste sind im Kohlenstoffgerüst jeweils geradkettig oder verzweigt. Wenn nicht speziell angegeben, sind bei diesen Resten die niederen Kohlenstoffgerüste, z.B. mit 1 bis 4 C-Atomen bzw. bei ungesättigten Gruppen mit 2 bis 4 C-Atomen, bevorzugt. Alkylreste, auch in den zusammengesetzten Bedeutungen wie Alkoxy, Haloalkyl usw., bedeuten, z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyle, 1-Methylhexyl und 1,4-Dimethylpentyl. Cycloalkyl bedeutet einen cycloaliphatischen Kohlenwasserstoffrest wie Cyclopropyl, Cyclobutyl, Cyclopentyl oder Cyclohexyl usw.; Alkenyl, Cycloalkenyl- und Alkinyl haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, Methylbut-3-en-1-yl und 1-Methyl-but-2-en-1-yl; Cycloalkenyl ist z.B. Cyclopentenyl und Cyclohexenyl; Alkinyl bedeutet z.B. Propargyl, But-2-in-1-yl, But-3-in-1-yl, 1-Methylbut-3-in-1-yl. Alkenyl in der Form "(C₃-C₄)Alkenyl" oder "(C₃-C₆)Alkenyl" bedeutet vorzugsweise einen Alkenylrest mit 3 bis 4 bzw. 3 bis 6 C-Atomen, bei dem die Doppelbindung nicht an dem C-Atom liegt, das mit dem übrigen Molekülteil der Verbindung verbunden ist ("yl"-Position). Entsprechendes gilt für (C₃-C₄)Alkinyl usw.
Halogen bedeutet beispielsweise Fluor, Chlor, Brom oder lod, Haloalkyl, -alkenyl und-alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl₂, CCl₃, CHCl₂, CH₂CH₂Cl; Haloalkyl ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechendes gilt für substituiertes Hydroxylamino oder Hydrazino.

Ein Acylrest bedeutet den Rest einer organischen Säure, z.B. den Rest einer Carbonsäure und Reste davon abgeleiteter Säuren wie der Thiocarbonsäure, gegebenenfalls N-substituierter Iminocarbonsäuren, oder der Rest von Kohlensäuremonoestern, gegebenenfalls N-substituierter Carbaminsäure, Sulfonsäuren, Sulfinsäuren, Phosphonsäuren, Phosphinsäuren. Acyl bedeutet beispielsweise Formyl, Alkylcarbonyl wie (C₁-C₄-Alkyl)-carbonyl, Phenylcarbonyl, wobei der Phenylring substituiert sein kann, z.B. wie oben für Phenyl gezeigt, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Die allgemeinen Formeln umfassen auch Stereoisomere, welche z. B. ein oder mehrere asymmetrische C-Atome oder auch Doppelbindungen enthalten, die in der jeweiligen allgemeinen Formel nicht gesondert angegeben sind. Die durch ihre spezifische Raumform definierten möglichen Stereoisomeren mit gleicher chemischer Verknüpfung, wie Enantiomere, Diastereomere, Z- und E-Isomere sind somit alle von der allgemeinen Formel umfaßt und können nach üblichen Methoden aus Gemischen der Stereoisomeren erhalten oder auch durch stereoselektiven Reaktionen in Kombination mit dem Einsatz von stereochemisch reinen Ausgangsstoffen hergestellt werden.

Die allgemeinen Formeln umfassen auch Tautomere der bezeichneten Verbindungen, soweit sie durch Protonenwanderung entstehen und soweit sie chemisch stabile Tautomere sind.

Die Verbindungen der Formel (I) können Salze bilden, bei denen der Wasserstoff der -SO₂-NH-Gruppe oder auch andere acide Wasserstoffatome (z.B. aus COOH u.a.) durch ein für die Landwirtschaft geeignetes Kation ersetzt wird. Diese Salze sind beispielsweise Metallsalze; vorzugsweise Alkali- oder Erdalkalisalze, insbesondere Natrium- und Kaliumsalze, oder auch Ammoniumsalze oder Salze mit organischen Aminen. Ebenso kann Salzbildung durch Anlagerung einer Säure an basische Gruppen, wie z.B. Amino und Alkylamino, erfolgen. Geeignete Säuren hierfür sind starke anorganische und organische Säuren, beispielsweise HCI, HBr, H₂SO₄ oder HNO₃.

Der organische Linker L in den Verbindungen der Formeln (II), (IV), (IV') und (IV") hat die Funktion einer Brücke zwischen dem Harzpolymer und dem Molekülteil mit der Sulfonamidocarbonylgruppe aus der Verbindung der Formel (III) bzw. Formel (I). Der Linker muß die Bindung des genannten Molekülteils und dessen spätere Abspaltung ermöglichen. Der Linker muß außerdem auf das Harzpolymer aufgebracht werden können, und zwar in der Regel durch eine chemische Reaktion, sofern das Harzpolymer nicht bereits aus geeigneten Monomeren, welche den Linker enthalten, aufgebaut werden kann.

Als Linker L eignen sich strukturell sehr unterschiedliche Reste, die in Abhängigkeit von den Bindungsstellen am Harzpolymer geeignete Bindungsstellen und funktionelle Gruppen aufweisen müssen. Überraschenderweise zeigen sich erfindungsgemäß sehr viele Linker als geeignet, die auch in der harzgebundenen Synthese für die Anbindung von Carbonsäuren, beispielsweise von Aminosäuren bei der Peptidsynthese, eingesetzt werden können.

Verbindungen (Linker-Komponenten), die für den Aufbau des Linkers in Kombination mit einem aminogruppenhaltigen Harz, z.B. einem Aminomethylenpolystyrolharz, oder einem hydroxygruppenhaltigen Harz eingesetzt werden können, sind Linker-Komponenten mit einer Carbonsäuregruppe. Die Herstellung der Harz-Linker-Verbindung der Formel (II) erfolgt dann jeweils durch Reaktion der Carboxygruppe einer Linker-Komponente mit einer Aminogruppe oder Hydroxygruppe des Harzes (Amidbildung bzw. Esterbildung).

Teilweise werden die Linker auch schrittweise hergestellt; in einem ersten Schritt wird eine Carbonsäure mit dem aminogruppenhaltigen Harz kondensiert und das erhaltene modifizierte Harz an den eingeführten Gruppen bis zur gewünschten Harz-Linker-Verbindung weiter modifiziert.

Des weiteren sind Harz-Linker-Verbindungen bekannt, welche auf Basis anderer Harze und auf andere Weise aufgebaut werden.

Beispiele für Linker-Komponenten und Harz-Linker-Verbindungen der Formel (II) sind nachfolgend in Tabelle 1 aufgeführt; die Linker-Komponente ist jeweils die Verbindung der Formel (V)

Z-L-Nuc (V),

worin Z die Abgangsgruppe oder zur Abgangsgruppe zu aktivierende funktionelle Gruppe ist, die bei der Umsetzung mit der Aminogruppe oder Hydroxygruppe des Harzes ersetzt wird; für den Fall, daß die Harz-Linker-Verbindung (II) anders hergestellt wird oder die Herstellung nicht detailliert angegeben ist, ist der Rest Z = "Polymer" angegeben, was die Bindungsstelle der funktionellen Gruppe -L-Nuc am Harzpolymer andeutet:

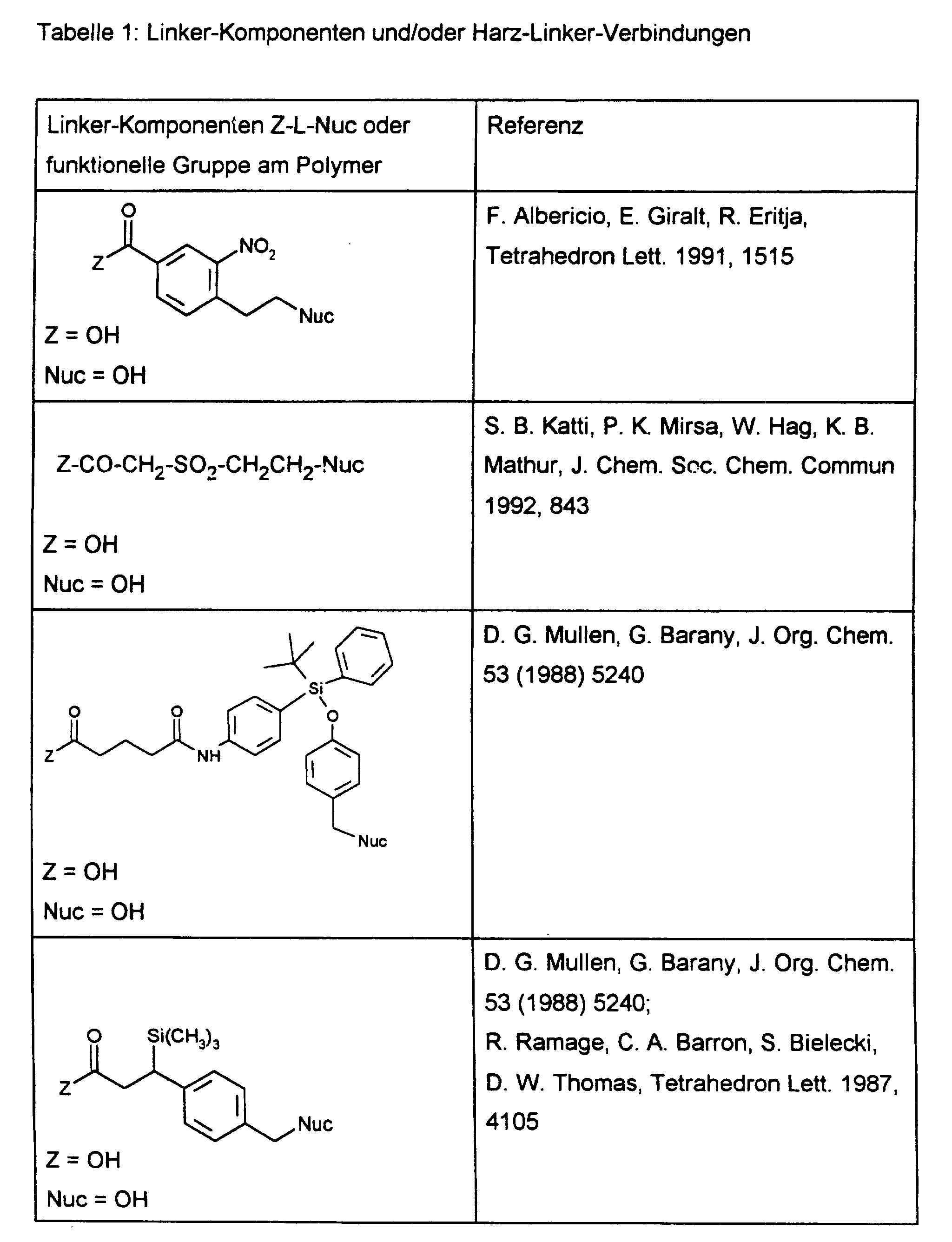

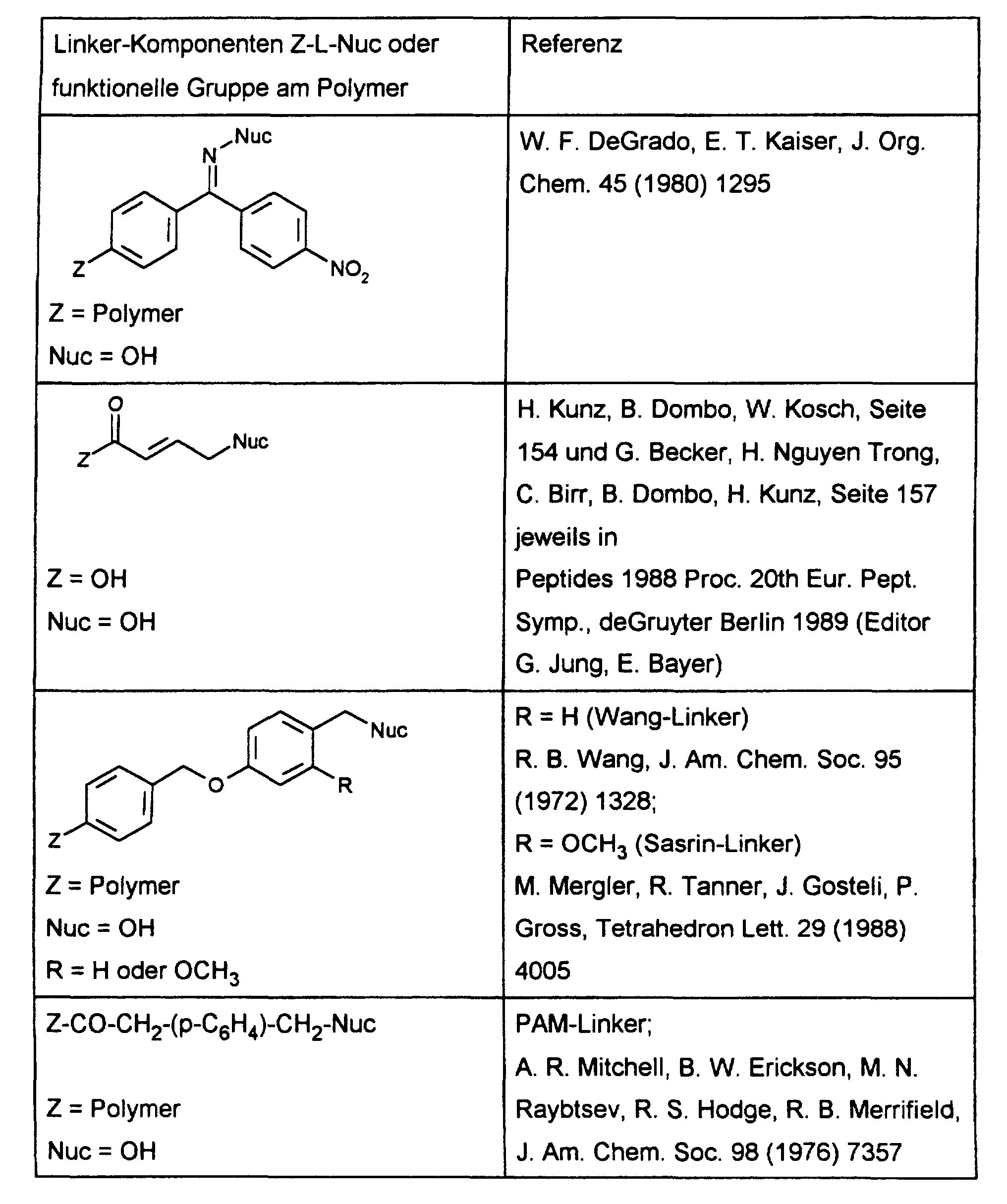

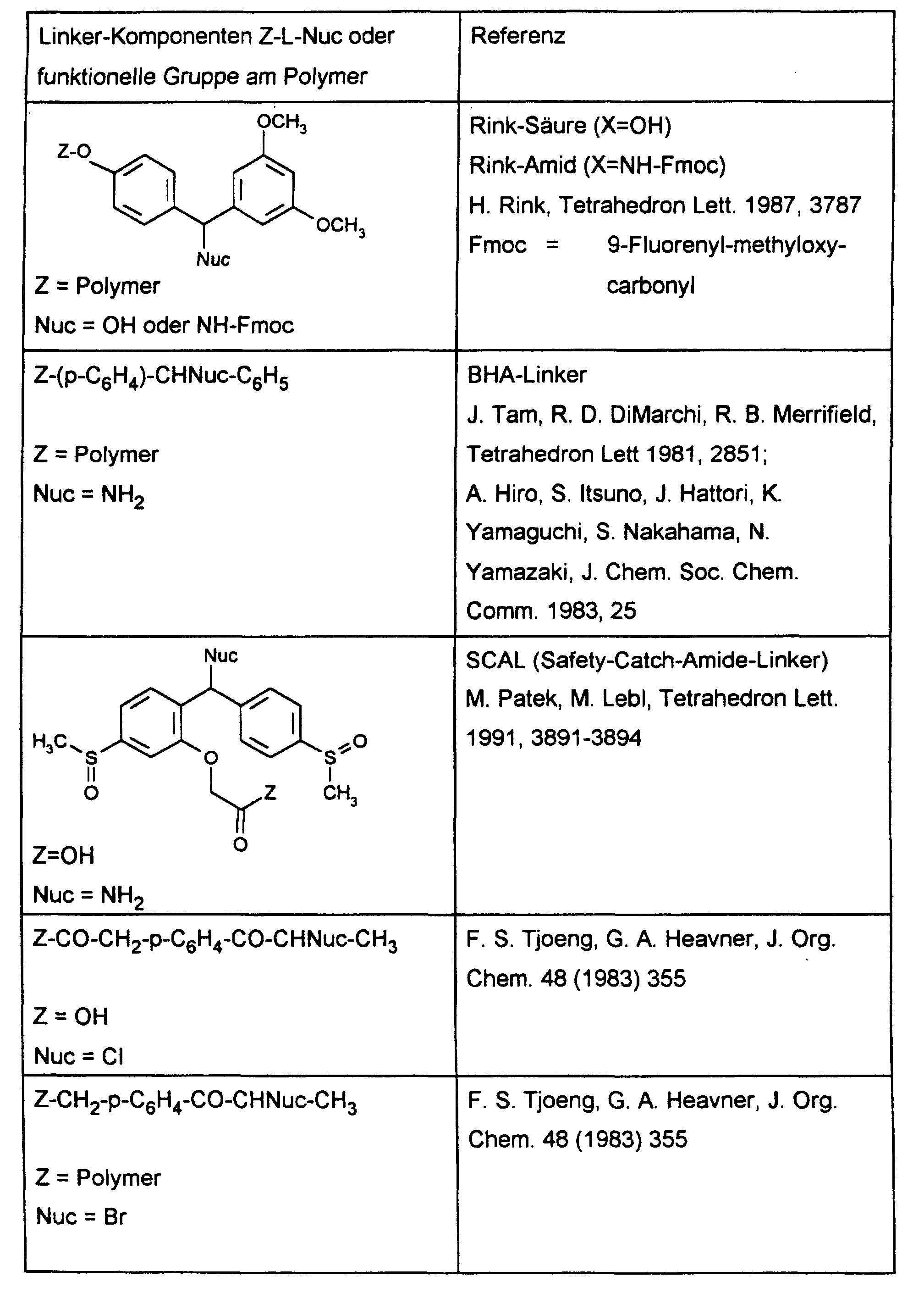

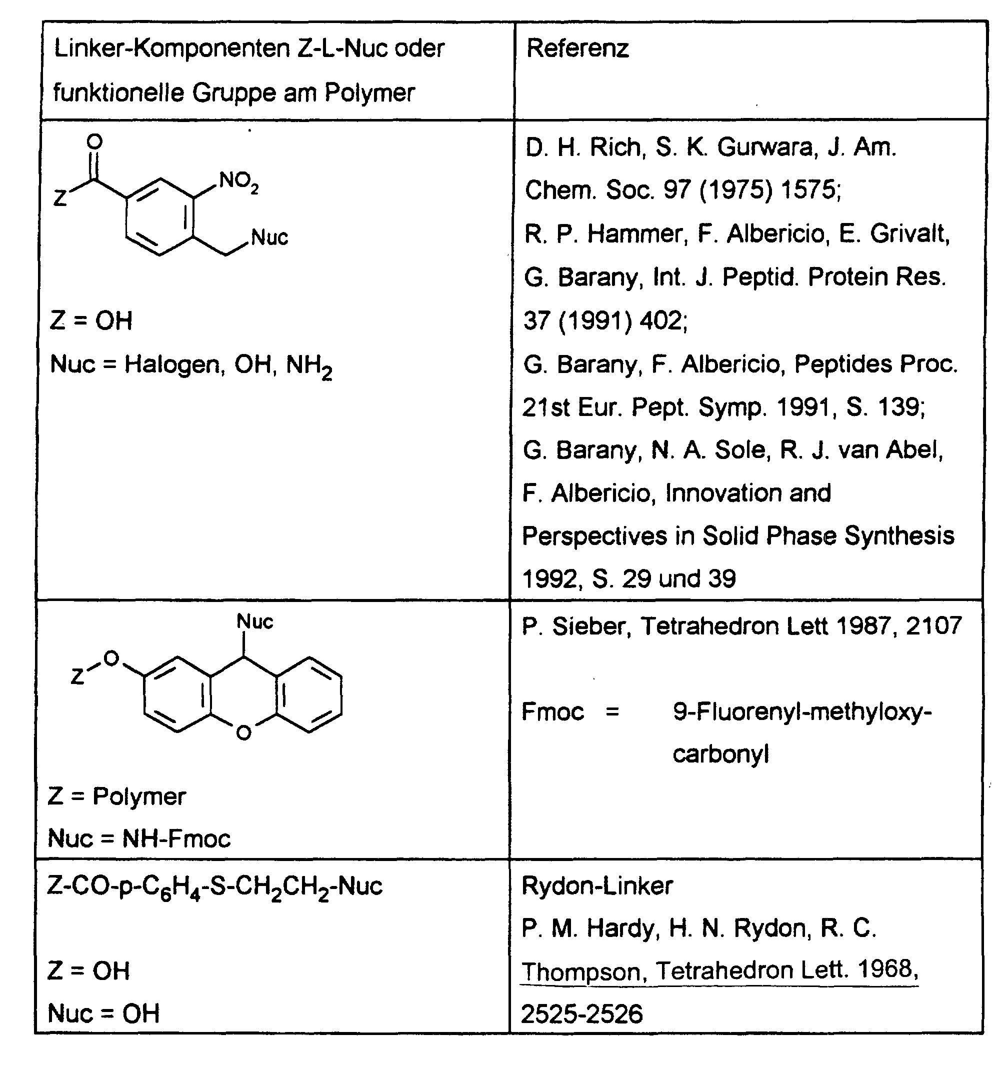

Die verwendbaren Harzpolymere sollen in den flüssigen Phasen, die für die Reaktionen und Isolierung der Verbindungen eingesetzt werden, unlöslich, weitgehend inert gegenüber den Reaktionsbedingungen in Stufe b) und filtrierbar sein; jedes Harzpolymerpartikel weist vorzugsweise viele Bindungsstellen für die jeweiligen Linker auf. In Abhängigkeit von der Struktur der gewählten Linker kommen vom Aufbau her gänzlich unterschiedliche Harzpolymere in Frage, beispielsweise Polystyrolharze, Polyamidharze, Polydimethylacrylamidharze, modifizierte Harze auf Basis der genannten Harze und Mischpolymere. Bevorzugte Harze sind Aminomethylenpolystyrolharze, d. h. aminomethylierte Polystyrolharze, oder auch anders modifizierte Harze auf Polystyrolbasis, z. B. Pfropfpolymerisate von Polystyrol und Polyethylenglykol wie solche aus der Reihe ®TentaGel (Fa. Rapp Polymere, Tübingen, Deutschland), in Form quellfähiger Partikel im Korngrößenbereich von beispielsweise 0,01 bis 1 mm, vorzugsweise 0,05 bis 0,5 mm, und einer Beladung von Aminomethylgruppen von 0,01 bis 10 mmol pro Gramm Harz, vorzugsweise 0,1 bis 2 mmol pro Gramm Harz.

Die einzelnen Linker werden in an sich bekannter Weise auf das Harz aufgebracht; siehe genannte Literatur in Tabelle 1. Dabei können unterschiedlichste Techniken eingesetzt werden. Geeignete Linker-Komponenten für die Kombination mit den aminomethylierten Polystyrolharzen sind der genannte Raydon-Linker und analoge Carbonsäureverbindungen. Linker wie der Raydon-Linker können unter den üblichen Bedingungen für Kondensationen und speziell für Amidbildungsreaktionen umgesetzt werden. Geeignet sind schonende Methoden bei moderaten Temperaturen. Die Umsetzung kann z. B. in einem weitgehend wasserfreien inerten organischen Lösungsmittel in Gegenwart von Katalysatoren oder üblichen Kondensationsmitteln bei Temperaturen von beispielsweise -30°C bis 200°C, vorzugsweise von 0°C bis 150°C, insbesondere 0°C bis 100°C durchgeführt werden.

Mit der Bezeichnung "inertes Lösungsmittel" sind Lösungsmittel gemeint, die unter den jeweiligen Reaktionsbedingungen inert sind, jedoch nicht unter beliebigen Reaktionsbedingungen inert sein müssen. Für die obengenannte Kondensation kommen beispielsweise folgende in Frage:
- Ether wie tert.-Butylmethylether, Dimethoxyethan (DME), Tetrahydrofuran (THF), Diethylether, Diisopropylether,
- dipolar aprotische Lösungsmittel wie Dimethylformamid (DMF), N,N-Dimethylacetamid (DMA), N-Methyl-pyrrolidon (NMP), Acetonitril,
- gegebenenfalls halogenierte aliphatische oder aromatische Kohlenwasserstoffe wie Dichlormethan, Toluol, o-Chlortoluol, Chlorbenzol, oder
- Gemische aus inerten Solventien.

Als Kondensationsmittel zur Herstellung der Harz-Linker-Verbindung (II) aus der Linker-Komponente und einem Aminomethylenpolystyrolharz eignen sich übliche Mittel wie azeotrope Destillation, Umsetzung mit aktivierten Derivaten der jeweiligen Linker-Carbonsäure wie Halogeniden oder aktiven Estern. Besonders geeignet sind schonende Methoden wie die Umsetzung in Gegenwart von Carbodiimiden wie Dicyclohexylcarbodiimid (DCC) oder Diisopropylcarbodiimid.

Die Umsetzung des Acylsulfonamids der Formel (III) mit der Harz-Linker-Verbindung (II) zu dem Harz-Linker-Addukt (IV) gelingt erfindungsgemäß durch einen Ersatz der nucleofugen Gruppe Nuc bzw. der bei der Austauschreaktion zu aktivierenden Gruppe Nuc durch die Amidogruppe der Sulfonylamidocarbonylgruppe in der Verbindung (III). Die Austauschreaktion kann im Falle einer Abgangsgruppe Nuc=Halogen oder Tosyl etc. mit der Verbindung der Formel (III) in Gegenwart von Basen erfolgen, bzw. analog den bei den bekannten Linkern verwendeten Austauschreaktionen.

Bevorzugt ist im Falle von Nuc = Hydroxyl, insbesondere bei Linkern wie dem Raydon-Linker eine Austauschreaktion unter Bedingungen wie sie analog für eine Mitsunobu-Reaktion (Red-Ox-Reaktion) angewendet werden, die für die Kondensation von Carbonsäuren oder anderen Elektrophilen mit Alkoholen mit Hilfe von Azodicarbonsäureestern und Triphenylphosphan bekannt ist; siehe z. B. O. Mitsunobu, Synthesis 1981, Seiten 1-28 und dort zitierte Literatur; siehe auch J. R. Henry et. al., Tetrahedron Lett. 1989, 5709-5712.

Die Reaktion verläuft nach dem Schema:

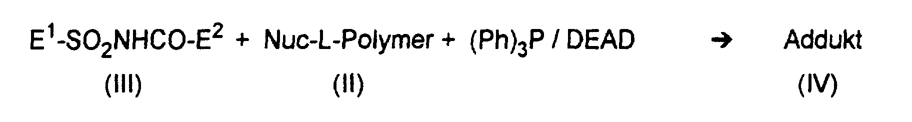

Dabei bedeutet Nuc eine Hydroxygruppe; Ph steht für Phenyl; DEAD steht für Azodicarbonsäurediethylester; andere Azodicarbonsäurediester sind ebenso verwendbar wie andere für die Durchführung der Mitsunobu-Reaktion verwendbare Reagenzien und Bedingungen. Die erfindungsgemäß angewandte Mitsunobu-Reaktion läßt sich in den bereits oben für die Kondensation der Linker-Komponente an das Harz genannten Lösungsmitteln bei moderaten Temperaturen, vorzugsweise unter wasserfreien neutralen Bedingungen bei 0°C bis 50°C durchführen.

Alternativ sind andere Kondensationsreaktionen möglich, wobei die jeweilige Methode von der Wahl der Abgangsgruppe Nuc abhängt.

Das Harz-Linker-Addukt (IV) ist erstaunlich stabil, auch bezüglich der Bindung zwischen Linker L und dem Stickstoffatom im Acylsulfonamid. Die Stabilität ermöglicht vielseitige chemische Umsetzungen an den Resten E¹ und E². Die Reste E¹ und E² müssen dabei so gewählt werden, daß eine Modifizierung der Reste zu den Resten R¹ bzw. R² in Formel (I) möglich ist.

Das Potential der Methode wird an folgender Synthesesequenz erläutert (vgl. Schema 1):

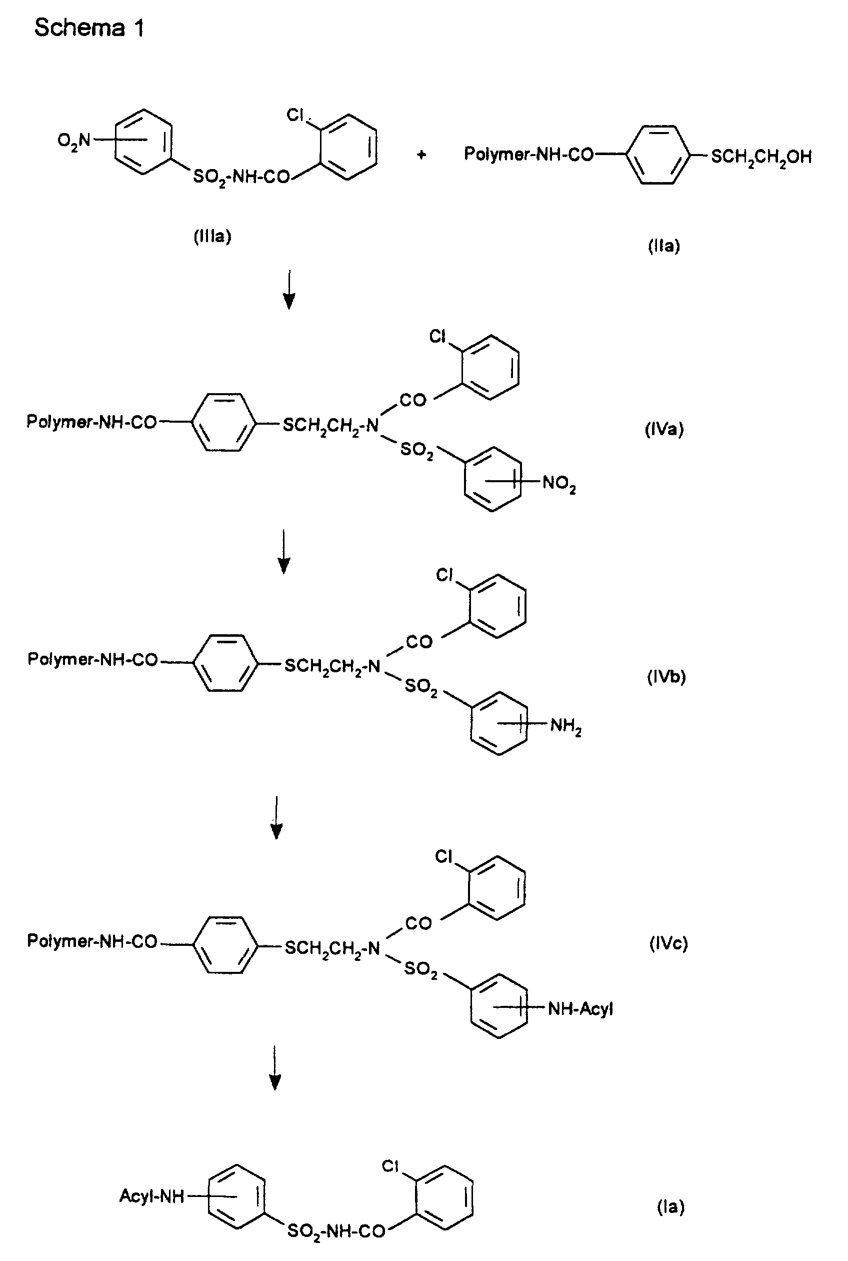

Gemäß Schema 1 wird nach der Anbindung des Acylsulfonamids der allgemeinen Formel (IIIa) an die Harz-Linker-Verbindung (lla) das Addukt (IVa) erhalten, welches an der Nitrogruppe zur Aminogruppe reduziert werden kann. Für die Reduktion eignen sich viele der für Nitrogruppen geeigneten chemischen Reduktionsmittel wie Metallsalze unter sauren Bedingungen, vorzugsweise milde Reduktionsmittel, die in organischen Lösungsmitteln eingesetzt werden können, wie Zinndichloriddihydrat/HCl, oder katalytische Reduktionen. Die erhaltene Aminoverbindung (IVb) läßt sich weiter modifizieren, z. B. durch (reduktive) Alkylierung oder durch Acylierung der Aminogruppe zur Verbindung der Formel (IVc). Die Acylierung kann wiederum mit einer Vielzahl von Acylierungsmitteln erfolgreich durchgeführt werden, beispielsweise mit Carbonsäurehalogeniden und Estern (vgl. Schema 1).

Die Abspaltung der herzustellenden Verbindung vom Harz erfolgt nach der für den einzelnen Linker typischen Reaktion oder Reaktionssequenz. Im Fall des Raydon-Linkers beinhaltet die Abspaltungsmethode die Oxidation des Schwefelatoms zum Sulfoxid oder zum Sulfon und die anschließende β-Eliminierung, wobei die Verbindung der Formel (I) freigesetzt und eine Ethenylsulfinyl- bzw. Ethenylsulfonylgruppe am Linker gebildet wird (vgl. Schema 1). Als Oxidationsmittel eignen sich chemische Oxidationsmittel für die Umwandlung von Thioethern in Sulfone, z. B. Peroxide wie Percarbonsäuren in organischen Lösungsmitteln, vorzugsweise solche wie m-Chlorperbenzoesäure in organischen Lösungsmitteln. Zur Beschleunigung der Eliminierung sind basische Bedingungen vorteilhaft. Beispielsweise kann man die Harzpartikel nach dem Oxidationsschritt mit einer basischen Lösung behandeln (Abspaltlösung), die im wesentlichen aus einem dipolar aprotischen Lösungsmittel, z. B. Ether wie Dioxan, Tetrahydrofuran (THF), 1,2-Dimethoxyethan oder 1,2-Diethoxyethan, besteht, dem geringe Mengen konzentrierter Natronlauge und gegebenenfalls weitere Lösungsvermittler, beispielsweise aus der Gruppe der Alkohole, wie Methanol, Ethanol, Propanol, Isopropanol oder tert.-Butanol, und Etheralkohole, wie Methoxyethanol, Methoxyethoxyethanol etc., zugesetzt werden. Im allgemeinen eignen sich auch wäßrige basische Bedingungen, vorzugsweise jedoch weitgehend wasserfreie Bedingungen unter Anwendung von Basen in organischen Lösungsmitteln.

Die Herstellung der Harz-Linker-Verbindung und die Reaktionen an ihr lassen sich mit erstaunlich vielen strukturellen Variationen bezüglich der Verbindungen der Formeln (III), (IV) und letztlich (I) durchführen. Die Reste der Formel E¹ oder E² sind im allgemeinen organische Reste, wie sie oben für R¹ und R² definiert worden sind.

Auf diese Weise ist es möglich, gezielt und in standardisierter Weise Wirkstoffstrukturen bereitzustellen und zu modifizieren. Einige der Verbindungen der allgemeinen Formel (la) und ihre Anwendung als Safener sind bereits in der deutschen Patentanmeldung Nr. 19621522.6 vorgeschlagen worden. Durch Variation der Ausgangsverbindungen der Formel (III) und der Derivatisierungsreaktionen ermöglicht die erfindungsgemäße Herstellungsmethode somit eine schnelle, systematische Herstellung von strukturvarianten Verbindungen, z. B. im Bereich der erwähnten Gruppe der Safener, welche phytotoxische Nebenwirkungen von Herbiziden reduzieren oder unterbinden können, oder im Bereich anderer chemischer Verbindungen, vor allem der Wirkstoffe für den Einsatz in der Humanmedizin, Tiermedizin oder im Pflanzenschutz.

Bevorzugte Reste für die Definitionen von R¹, R², E¹, E², (E¹)' und (E²)' sind: Aliphatische oder aromatische Kohlenwasserstoffreste oder Kohlenwasserstoffoxyreste wie Alkyl, Alkoxy, Alkenyl, Alkenyloxy, Alkinyl, Alkinyloxy, Cycloalkyl, Cycloalkoxy, Aryl, vorzugsweise Phenyl, oder Aryloxy oder heterocyclische Reste, wobei jeder der vorstehenden Reste jeweils unsubstituiert oder durch einen oder mehrere Reste aus der Gruppe Halogen, Hydroxy, Amino, mono- und disubstituiertes Amino, Nitro, Cyano, Cyanato, Thiocyanato, Azido, Alkyl, Cycloalkyl, Alkenyl, Cycloalkenyl, Alkinyl, Alkoxy, Alkylthio, Alkenyloxy, Cycloalkoxy, Cycloalkenyloxy und Alkinyloxy, wobei jeder der letztgenannten 15 Reste unsubstituiert oder durch einen oder mehrere Reste aus der Gruppe Halogen, CN, NO₂, Alkoxy, Alkylthio, Haloalkoxy, Acyl, Acyloxy, Amino, mono- und disubstituiertes Amino und im Falle cyclischer Reste auch Alkyl und Haloalkyl substituiert ist, und unsubstituiertes und substituiertes Aryl, unsubstituiertes und substituiertes Aryloxy, unsubstituiertes und substituiertes Heterocyclyl, unsubstituiertes und substutiertes Heterocyclyloxy, Acyl und Acyloxy substituiert ist.

Weiterhin von Interesse sind erfindungsgemäße Verfahren zur Herstellung von Verbindungen der Formel (I), worin
- R¹: (C₁-C₆)Alkyl, (C₃-C₆)Cycloalkyl, (C₂-C₆)Alkenyl, (C₅-C₆)Cycloalkenyl oder (C₂-C₆)Alkinyl, wobei jeder der letztgenannten 5 Reste unsubstituiert oder durch einen oder mehrere Reste aus der Gruppe Halogen, (C₃-C₆)Cycloalkyl, (C₁-C₄)Alkoxy, (C₁-C₄)Alkylthio, (C₁-C₄)Haloalkoxy, unsubstituiertes und substituiertes Aryl, unsubstituiertes und substituiertes Heterocyclyl, unsubstituiertes und substitutiertes Aryloxy und im Falle cyclischer Reste auch (C₁-C₄)Alkyl und (C₁-C₄)Haloalkyl substituiert ist, oder
Phenyl oder Heterocyclyl, wobei jeder der beiden letztgenannten Reste unsubstituiert oder durch einen oder mehrere Reste aus der Gruppe Halogen, Nitro, Cyano, Thiocyanato, Amino, mono- und disubstituiertes Amino, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₅)Alkanoyl, (C₁-C₄)Alkoxy-carbonyl und Phenylcarbonyl, wobei jeder der letztgenannten 8 Reste unsubstituiert oder durch einen oder mehrere Reste aus der Gruppe Halogen, (C₁-C₄)Alkoxy, (C₁-C₄)Alkylthio, (C₁-C₄)Haloalkoxy und im Falle cyclischer Reste auch (C₁-C₄)Alkyl und (C₁-C₄)Haloalkyl substituiert ist, substituiert ist, und
- R²: (C₁-C₆)Alkyl, (C₃-C₆)Cycloalkyl, (C₂-C₆)Alkenyl, (C₅-C₆)Cycloalkenyl, (C₂-C₆)Alkinyl, (C₁-C₆)Alkoxy, (C₁-C₆)Alkylthio, (C₃-C₆)Alkenyloxy, (C₃-C₆)Cycloalkoxy, (C₅-C₆)Cycloalkenyloxy oder (C₃-C₆)Alkinyloxy, wobei jeder der letztgenannten 11 Reste unsubstituiert oder durch einen oder mehrere Reste aus der Gruppe Halogen, (C₃-C₆)Cycloalkyl, (C₁-C₄)Alkoxy, (C₁-C₄)Alkylthio, (C₁-C₄)Haloalkoxy, unsubstituiertes und substituiertes Aryl, unsubstituiertes und substituiertes Heterocyclyl, unsubstituiertes und substutiertes Aryloxy und im Falle cyclischer Reste auch (C₁-C₄)Alkyl und (C₁-C₄)Haloalkyl substituiert ist, oder
Phenyl, Phenoxy, Heteroaryl oder Heteroaryloxy, wobei jeder der vier letztgenannten Reste unsubstituiert oder durch einen oder mehrere Reste aus der Gruppe Halogen, Nitro, Cyano, Thiocyanato, Amino, mono- und disubstituiertes Amino, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₅)Alkanoyl, (C₁-C₄)Alkoxycarbonyl und Phenylcarbonyl, wobei jeder der letztgenannten 8 Reste unsubstituiert oder durch einen oder mehrere Reste aus der Gruppe Halogen, (C₁-C₄)Alkoxy, (C₁-C₄)Alkylthio, (C₁-C₄)Haloalkoxy und im Falle cyclischer Reste auch (C₁-C₄)Alkyl und (C₁-C₄)Haloalkyl substituiert ist, substituiert ist,
bedeuten.

Weiterhin bevorzugt sind erfindungsgemäße Herstellungen zu Verbindungen der Formel (I), worin
- R¹: (C₁-C₆)Alkyl, das unsubstituiert oder durch einen oder mehrere Reste aus der Gruppe Halogen, (C₃-C₆)Cycloalkyl, (C₁-C₄)Alkoxy, (C₁-C₄)Alkylthio, (C₁-C₄)Haloalkoxy, Phenyl und Heteroaryl, wobei jeder der beiden letztgenannten Reste unsubstituiert oder durch einen oder mehrere Reste aus der Gruppe Halogen, Nitro, Cyano, (C₁-C₄)Alkyl, (C₁-C₄)Haloalkyl, (C₁-C₄)Alkoxy, (C₁-C₄)Alkylthio und (C₁-C₄)Haloalkoxy, substituiert ist, substituiert ist, oder (C₃-C₆)Cycloalkyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio und (C₁-C₄)Haloalkoxy substituiert ist, oder
Phenyl oder Heteroaryl, wobei jeder der beiden letztgenannten Reste unsubstituiert oder durch einen oder mehrere Reste aus der Gruppe Halogen, Nitro, Cyano, Thiocyanato, Amino, Mono- und Di-[(C₁-C₄)Alkyl]amino, Acylamino, N-Acyl-N-(C₁-C₄)alkyl-amino, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₅)Alkanoyl, (C₁-C₄)Alkoxy-carbonyl und Phenylcarbonyl, wobei jeder der letztgenannten 8 Reste unsubstituiert oder durch einen oder mehrere Reste aus der Gruppe Halogen, (C₁-C₄)Alkoxy, (C₁-C₄)Alkylthio, (C₁-C₄)Haloalkoxy und im Falle cyclischer Reste auch (C₁-C₄)Alkyl und (C₁-C₄)Haloalkyl substituiert ist, substituiert ist, und
- R²: (C₁-C₆)Alkyl oder (C₁-C₆)Alkoxy, wobei jeder der beiden letztgenannten Reste unsubstituiert oder durch einen oder mehrere Reste aus der Gruppe Halogen, (C₃-C₆)Cycloalkyl, (C₁-C₄)Alkoxy, (C₁-C₄)Alkylthio, (C₁-C₄)Haloalkoxy, Phenyl, Phenoxy und Heteroaryl, wobei jeder der drei letztgenannten Reste unsubstituiert oder durch einen oder mehrere Reste aus der Gruppe Halogen, Nitro, Cyano, (C₁-C₄)Alkyl, (C₁-C₄)Haloalkyl, (C₁-C₄)Alkoxy, (C₁-C₄)Alkylthio und (C₁-C₄)Haloalkoxy, substituiert ist, substituiert ist, oder
(C₃-C₆)Cycloalkyl, (C₂-C₆)Alkenyl, (C₂-C₆)Alkinyl, (C₁-C₆)Alkylthio, (C₃-C₆)Alkenyloxy, (C₃-C₆)Cycloalkoxy oder (C₃-C₆)Alkinyloxy oder
Phenyl, Phenoxy, Heteroaryl oder Heteroaryloxy, wobei jeder der vier letztgenannten Reste unsubstituiert oder durch einen oder mehrere Reste aus der Gruppe Halogen, Nitro, Cyano, Thiocyanato, Amino, Mono- und Di[(C₁-C₄)Alkyl]-amino, Acylamino, N-Acyl-N-(C₁-C₄)alkyl-amino, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₅)Alkanoyl, (C₁-C₄)Alkoxy-carbonyl und Phenylcarbonyl, wobei jeder der letztgenannten 8 Reste unsubstituiert oder durch einen oder mehrere Reste aus der Gruppe Halogen, (C₁-C₄)Alkoxy, (C₁-C₄)Alkylthio und (C₁-C₄)Haloalkoxy und im Falle cyclischer Reste auch (C₁-C₄)Alkyl und (C₁-C₄)Haloalkyl substituiert ist, substituiert ist, bedeuten.

Insbesondere bevorzugt sind erfindungsgemäße Herstellungen zu Verbindungen der Formel (I), worin
- R¹: (C₁-C₄)Alkyl, (C₁-C₄)Haloalkyl, (C₃-C₆)Cycloalkyl, das unsubstituiert oder durch einen oder mehrere Reste aus der Gruppe Halogen, (C₁-C₄)Alkyl und (C₁-C₄)Haloalkyl substituiert ist, oder
Phenyl, das unsubstituiert oder durch einen oder mehrere Reste aus der Gruppe Halogen, Nitro, Cyano, Amino, Mono- und Di-[(C₁-C₄)Alkyl]-amino, Acylamino, N-Acyl-N-(C₁-C₄)alkyl-amino, (C₁-C₄)Alkyl, (C₁-C₄)Alkoxy und (C₁-C₄)Alkylthio substituiert ist, und
- R²: (C₁-C₄)Alkylthio, oder (C₁-C₄)Alkanoyl, wobei jeder der beiden letztgenannten Reste unsubstituiert oder durch einen oder mehrere Reste aus der Gruppe Halogen, (C₃-C₆)Cycloalkyl, (C₁-C₄)Alkyl, (C₁-C₄)Alkylthio und (C₁-C₄)Haloalkoxy substituiert ist, oder
Phenyl, das unsubstituiert oder durch einen oder mehrere Reste aus der Gruppe Halogen, Nitro, Cyano, Amino, Mono- und Di-[(C₁-C₄)Alkyl]-amino, Acylamino, N-Acyl-N-(C₁-C₄)alkyl-amino, (C₁-C₄)Alkyl, (C₁-C₄)Alkoxy, (C₁-C₄)Alkylthio (C₁-C₄)Alkylsulfinyl, (C₁-C₄)Alkylsulfonyl, (C₁-C₅)Alkanoyl, (C₁-C₄)Alkoxy-carbonyl und Phenylcarbonyl substituiert ist, bedeuten.

Weiterhin besonders bevorzugt sind erfindungsgemäße Herstellungen zu Verbindungen der Formel (I), worin
- R¹: Phenyl, das unsubstituiert oder durch einen Rest aus der Gruppe Nitro, Amino, Mono- und Di-[(C₁-C₄)Alkyl]-amino, (C₁-C₄)Alkylcarbonylamino, (C₁-C₄)Alkoxy-carbonylamino, Mono- und Di-[(C₁-C₄)Alkyl]-aminocarbonylamino substituiert ist, und
- R²: (C₁-C₄)Alkoxy oder
Phenyl, das unsubstituiert oder durch einen oder mehrere Reste aus der Gruppe Halogen, Nitro, Cyano, (C₁-C₄)Alkyl, (C₁-C₄)Alkoxy und (C₁-C₄)Alkylthio substituiert ist, bedeuten.

Den Verbindungen der Formel (I) mit den Resten R¹ und R² entsprechen Verbindungen der Formel (IV), (IV') und (IV') mit entsprechenden Resten bzw. entsprechend geeigneten Vorstufen der Reste.

Viele der hergestellten und der herstellbaren Verbindungen der Formel (I) sind neu. Insbesondere sind Verbindungen der Formel (I) neu, worin
- R¹: Phenyl, das in meta- oder ortho-Position mit einem Rest der Formel Mono- oder Di-[(C₁-C₄)Alkyl]-amino, (C₁-C₄)Alkylcarbonylamino, (C₁-C₄)Alkoxycarbonylamino, Mono- oder Di-[(C₁-C₄)Alkyl]-aminocarbonylamino substituiert ist, und
- R²: (C₁-C₄)Alkoxy oder
Phenyl, das unsubstituiert oder durch einen oder mehrere Reste aus der Gruppe Halogen, Nitro, Cyano, (C₁-C₄)Alkyl, (C₁-C₄)Alkoxy und (C₁-C₄)Alkylthio substituiert ist, bedeuten.

Diese Verbindungen sind durch das erfindungsgemäße Verfahren in systematischer Weise zugänglich und eignen sich teilweise als Wirkstoffe für den Pflanzenschutz und als wertvolle Zwischenprodukte zur Herstellung biologisch aktiver Verbindungen.

In den folgenden Beispielen beziehen sich Mengenangaben auf das Gewicht, sofern nichts anderes angegeben ist. In den Beispielen verwendete Abkürzungen sind dem Fachmann geläufig oder werden teilweise an anderer Stelle der Beschreibung erläutert.

### Beispiele

### 1. Herstellung der Harz-Linker-Verbindung

### 1a) Herstellung des Linkers 4-(2-Hydroxyethylthio)-benzoesäure

2,0 I Methylenchlorid wurden unter Stickstoffatmosphäre mit 46,92 g (304 mmol) 4-Mercaptobenzoesäure versetzt. Unter Stickstoffatmosphäre wurden 57,0 g (456 mmol) 2-Bromethanol und 61,5 g (609 mmol) Triethylamin getropft. Die Lösung wurde über Nacht bei Raumtemperatur gerührt. Anschließend filtrierte man wenig ungelösten Feststoff ab, engte das Filtrat ein und nahm den Rückstand in 500 ml 2n NaOH auf. Diese Phase wurde einmal mit Ether extrahiert. Anschließend säuerte man die wässrige Phase auf pH=4 an und isolierte das ausgefallene Produkt durch Filtration. Nach Kristallisation aus Acetonitril erhielt man das unter Titel 1a) definierte Produkt; Ausbeute: 34,21 g (56,7 %); Schmp.: 150°C.

### 1b) Herstellung der Harz-Linker-Verbindung N-(4-Hydroxyethylthio-benzoyl)-aminomethylenpolystyrolharz

20,0 g Aminomethylen-polystyrolharz (1,07 mmol Aminofunktion pro Gramm Harz) wurden mit 6,36 g (32,1 mmol) 4-(Hydroxyethylthio)benzoesäure und 2,89 g (21,4 mmol) 1-Hydroxybenzotriazol in 200 ml wasserfreiem Tetrahydrofuran (THF) suspendiert. Unter Stickstoffatmosphäre wurden 8,10 g (64,2 mmol) Diisopropylcarbodiimid zugegeben. Die Suspension stand für 64 h bei Raumtemperatur und wurde anschließend filtriert.
Das Produkt wusch man je fünfmal mit je 100 ml Dimethylformamid (DMF) und Methanol, anschließend mehrfach mit THF und abschließend mit Ether. Das erhaltene Harz wurde im Exsiccator getrocknet. Man erhielt das unter dem Titel 1b) definierte Harz-Linker-Addukt mit einer Rohausbeute von 24,8 g (104 % d. Th.).

### 2. Herstellung des Harz-Linker-Addukts mit einem Sulfonamid

### 2a) Herstellung von N-(2-Chlorbenzoyl)-3-nitrophenylsulfonamid

20,0 g (98,9 mmol) 3-Nitrophenylsulfonamid wurden zusammen mit 243 mg (1,98 mmol) 4-N,N-Dimethylaminopyridin in 300 ml wasserfreiem Acetonitril vorgelegt. Nach Zugabe von 23,0 g (228 mmol) Triethylamin wurde auf 0°C abgekühlt und 19,04 g (108,8 mmol) 2-Chlorbenzoylchlorid zugetropft. Nach Entfernen der Kühlung erwärmte sich die Reaktionslösung innerhalb von 4 h auf Raumtemperatur. Die Lösung wurde unter Vakuum eingeengt und der Rückstand in Essigester aufgenommen. Die organische Phase extrahierte man zweimal mit 2n HCI, wusch mit Wasser und trocknete über Magnesiumsulfat. Nach Abfiltrieren und Entfernen des Lösungsmittels wurde das Rohprodukt aus Toluol kristallisiert. Man erhielt N-(2-Chlorbenzoyl)-3-nitrophenylsulfonamid in einer Ausbeute von 31,2 g (92,6%); Schmp.: 135°C.

### 2b) Herstellung des Addukts N-{4-[N-(2-Chlorbenzoyl)-N-(3-nitrophenylsulfonyl)-aminoethylthio]-benzoyl}aminomethylenpolystyrolharz

In einer Lösung von 9,17 g (26,9 mmol) N-(2-Chlorbenzoyl)-3-nitrophenylsulfonamid und 9,40 g (35,9 mmol) Triphenylphosphan wurden 10,0 g (8,97 mmol Hydroxyfunktion) N-(4-Hydroxyethylthio-benzoyl)-aminomethylenpolystyrolharz suspendiert. Unter Stickstoffatmosphäre gab man 3,96 g (31,4 mmol) Azodicarbonsäurediethylester zu und rührte 6 h bei Raumtemperatur. Zur Aufarbeitung wurde filtriert, je dreimal mit 100 ml Dimethylformamid, THF und Diethylether gewaschen. Das erhaltene, unter Titel 2b) definierte Harz wurde über Nacht im Exsiccator getrocknet; Rohausbeute: 13,62 g (105,6 % d. Th.).

### 3. Umsetzungen mit dem Harz-Linker-Addukt

### 3.1 Reduktion der Nitrogruppe zum Produkt N-{4-[N-(2-Chlorbenzoyl)-N-(3-aminophenyl-sulfonyl)-aminoethylthio]-benzoyl}-aminomethylenpolystyrolharz

1,57 g (6,96 mmol) Zinndichloriddihydrat wurden in 45 ml Dimethylformamid gelöst und mit 2,5 ml konzentrierter Salzsäure versetzt. Anschließend wurde 1,00 g (0,70 mmol Nitrofunktion) N-{4-[N-(2-chlorbenzoyl)-N-(3-nitrophenylsulfonyl)-aminoethylthio]-benzoyl}-aminomethylenpolystyrolharz zugefügt und 4 h bei 50-55°C gerührt, anschließend abgekühlt und filtriert. Das erhaltene Harz wusch man je dreimal mit je 20 ml Dimethylformamid, THF, Dioxan, Methylenchlorid und abschließend mit Diethylether. Nach Trocknen des Produktes erhielt man das Titel-Produkt (3.1) mit der Rohausbeute von 1,05 g (107,2 % d. Th.).

### 3.2 Acylierung der Aminofunktion und Abspaltung zu N-(2-Chlorbenzoyl)-3-(isopropylcarbonylamino)-benzolsulfonamid

0,300 g (0,210 mmol Aminofunktion) N-{4-[N-(2-Chlorbenzoyl)-N-(3-aminophenylsulfonyl)-aminoethylthio]-benzoyl}-aminomethylenpolystyrolharz wurden in 30 ml Methylenchlorid suspendiert, auf -10°C unter Stickstoffatmosphäre gekühlt und mit 227 mg (2,13 mmol) Isopropylcarbonylchlorid versetzt. Anschließend gab man unter Rühren 26 mg (0,21 mmol) 4-N,N-Dimethylaminopyridin und 215 mg (2,13 mmol) Triethylamin zu. Die Suspension stand über Nacht bei Raumtemperatur und wurde anschließend filtriert. Das erhaltene acylierte Harz wusch man je dreimal mit je 20 ml Methylenchlorid, Dimethylformamid, THF und Diethylether. Anschließend wurde das acylierte Harz in 10 ml Methylenchlorid suspendiert und mit 410 mg (1,66 mmol) 70%iger 3-Chlorperbenzoesäure 10 min oxidiert. Die Suspension wurde filtriert und je dreimal mit 20 ml Methylenchlorid und Dioxan gewaschen. Daraufhin setzte man 4 ml Abspaltungslösung zu (Abspaltlösung: 25 ml Dioxan, 3 ml 2-Methoxyethanol, 0,5 ml 5n NaOH in H₂O) und ließ 10 min reagieren. Die Lösung wurde abfiltriert und der Vorgang wiederholt. Die vereinigten Filtrate wurden über 1,5 g saurem Aluminiumoxid filtriert. Das Aluminiumoxid wusch man zweimal mit einer Lösung aus Dioxan/Ethanol (7:3) nach. Die vereinigten Filtrate engte man bis zur Trockene ein und erhielt 79,1 mg (92%) des gewünschten Titel-Produkts; Schmp. 177°C (Zers.); ¹H-NMR (DMSO-d₆, TMS): δ =1.12 (d, J=5.5Hz, 6H, CH-(CH₃)₂), 2.64 (sept., J=5.5 Hz, 1 H, CH-(CH₃)₂), 7.35-7.65 (m, 6H, aromatisches H), 7.90 (m, 1H, aromatisches H), 8.40 (m, 1H, aromatisches H), 10.20 (s, 1H, NH-CO-CH), 12.40 (s, 1H, SO₂-NH-CO).

### 4. Herstellung des Sulfonamids N-Methoxycarbonyl-3-nitrophenylsulfonamid

4,40 g (108 mmol) Natriumhydrid (60%ig in Oel) wurden in 250 ml wasserfreiem THF suspendiert. Die Suspension versetzte man zunächst mit einer Lösung von 10,0 g (49,5 mmol) 3-Nitrophenylsulfonamid in 100 ml wasserfreiem Tetrahydrofuran und anschließend mit einer Lösung von 5,60 g (54,4 mmol) Chlorameisensäuremethylester. Nach 100 h Rühren bei Raumtemperatur wurde auf Eiswasser gegeben, mit 2n HCI angesäuert und dreimal mit Essigester gewaschen, anschließend über Natriumsulfat getrocknet, filtriert und eingeengt. Das Rohprodukt kristallisierte man aus Essigester / n-Heptan und erhielt N-Methoxycarbonyl-3-nitrophenylsulfonamid mit einer Ausbeute von 8,64 g (67%); Schmp.: 121-123°C.

### 5. Herstellung des Harz-Linker-Addukts N-{4-[N-(Methoxycarbonyl)-N-(3-nitrophenylsulfonyl)-aminoethylthio]-benzoyl}-aminomethylenpolystyrolharz

3,00 g (2,69 mmol Hydroxyfunktion) 4-Hydroxyethylthio-benzoylaminomethylenpolystyrolharz wurden unter N₂-Atmosphäre in 100 ml THF suspendiert und anschließend mit 2,10 g (8,07 mmol) N-Methoxycarbonyl-3-nitrobenzolsulfonamid und 2,82 g (10,8 mmol) Triphenylphosphan versetzt. Zu dieser Suspension tropfte man 1,19 g (9,42 mmol) Azodicarbonsäure-diethylester. Nach 16 h Stehenlassen bei Raumtemperatur wurde das Harz abfiltriert und je dreimal mit je 50 ml Dimethylformamid, Tetrahydrofuran und Diethylether gewaschen. Nach Trocknen im Exsiccator erhielt man das Titel-Produkt (5.) mit einer Rohausbeute von 3,89 g (107%).

### 6. Reduktion der Nitrogruppe am Harz-Linker-Addukt zum Produkt N-{4-[N-(Methoxycarbonyl)-N-(3-aminophenylsulfonyl)-aminoethylthio]-benzoyl}-aminomethylenpolystyrolharz

3,59 g (26,5 mmol) N-{4-[N-(Methoxycarbonyl)-N-(3-nitrophenylsulfonyl)-aminoethylthio]-benzoyl}-aminomethylenpolystyrolharz wurden in einer Lösung von 5,95 g (26,5 mmol) Zinndichloriddihydrat, 4,20 ml konzentrierter Salzsäure in 40 ml Dimethylformamid 4 h auf 55°C erwärmt. Das Harz wurde abfiltriert und je dreimal mit 20 ml Dimethylformamid, THF und Ether gewaschen. Nach Trocknen im Exsiccator erhielt man das Rohprodukt der Titelverbindung (6.); Rohausbeute: 3,63 g (103%).

### 7. Acylierung und Abspaltung zu N-(Methoxycarbonyl)-3-(isopropylcarbonylamino)-benzolsulfonamid

300 mg (230 µmol Aminofunktion) N-{4-[N-(Methoxycarbonyl)-N-(3-aminophenylsulfonyl)-aminoethylthio]-benzoyl}-aminomethylenpolystyrolharz wurden unter Stickstoffatmosphäre in 6 ml Methylenchlorid suspendiert, auf 0°C gekühlt und mit 241 mg (2,26 mmol) Isopropylcarbonylchlorid versetzt. Anschließend gab man unter Rühren 28 mg (0,23 mmol) 4-N,N'-Dimethylaminopyridin und 229 mg (2,26 mmol) Triethylamin zu. Die Suspension stand über Nacht bei Raumtemperatur und wurde anschließend filtriert. Das Harz wusch man mit je 25 ml Methylenchlorid, Dimethylformamid, THF und Ether. Im Anschluß wurde das acylierte Harz in 4 ml Methylenchlorid suspendiert und mit 410 mg (1,66 mmol) 70%iger 3-Chlorperbenzoesäure 10 min oxidiert. Die Suspension wurde erneut filtriert und je dreimal mit 4 ml Methylenchlorid und Dioxan gewaschen. Anschließend setzte man 4 ml einer Abspaltlösung zu (Abspaltlösung: 25 ml Dioxan, 3 ml 2-Methoxyethanol, 0,5 ml 5n NaOH in H₂O) und ließ 10 min reagieren. Die Abspaltlösung wurde abfiltriert und der Vorgang wiederholt. Die vereinigten Filtrate filtrierte man über 1,5 g saurem Aluminiumoxid. Das Aluminiumoxid wusch man mit Dioxan/Ethanol (7:3) nach. Die vereinigten Filtrate wurden bis zur Trockene eingeengt und man erhielt N-(Methoxycarbonyl)-3-(isopropylcarbonylamino)-benzolsulfonamid mit einer Ausbeute von 67,4 mg (97,6% d. Th.); ¹H-NMR (DMSO-d₆, TMS): δ = 1.10 (d, J=6.0 Hz, 6H, CH-(CH₃)₂), 2.65 (sept., J=6.0 Hz, 1 H, CH-(CH₃)₂), 3.55 (s, 3H, O-CH₃), 7.48 (m, 2H, aromatisches H), 7.85 (m, 1H, aromatische H), 8.20 (m, 1H, aromatisches H), 10.20 (s, 1H, NH-CO-CH), 12.20 (s, 1H, SO₂-NH-CO).

Auf analoge Weise zu den Beispielen 1 bis 7 wurden die in der folgenden Tabelle 2 aufgeführten Verbindungen hergestellt.

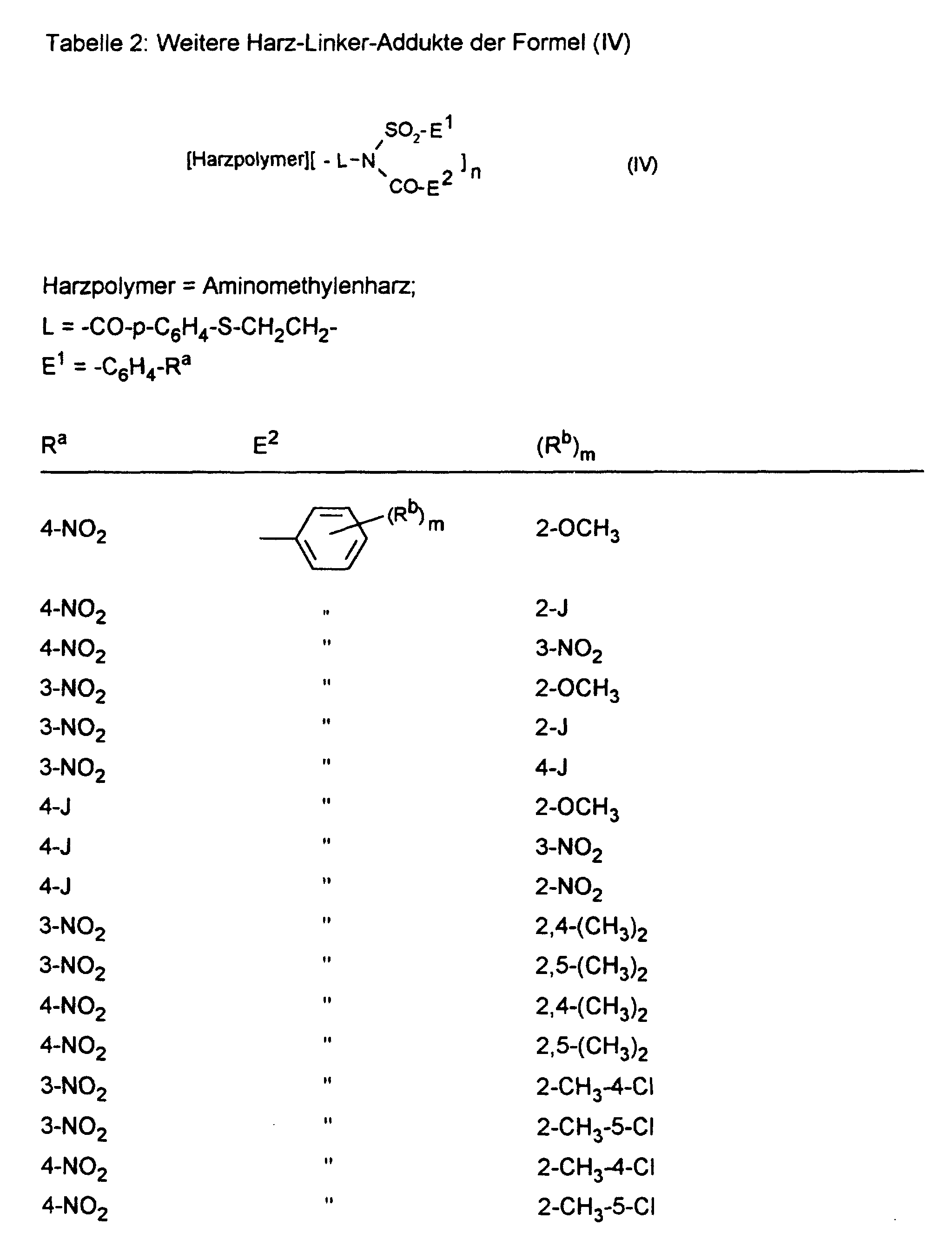

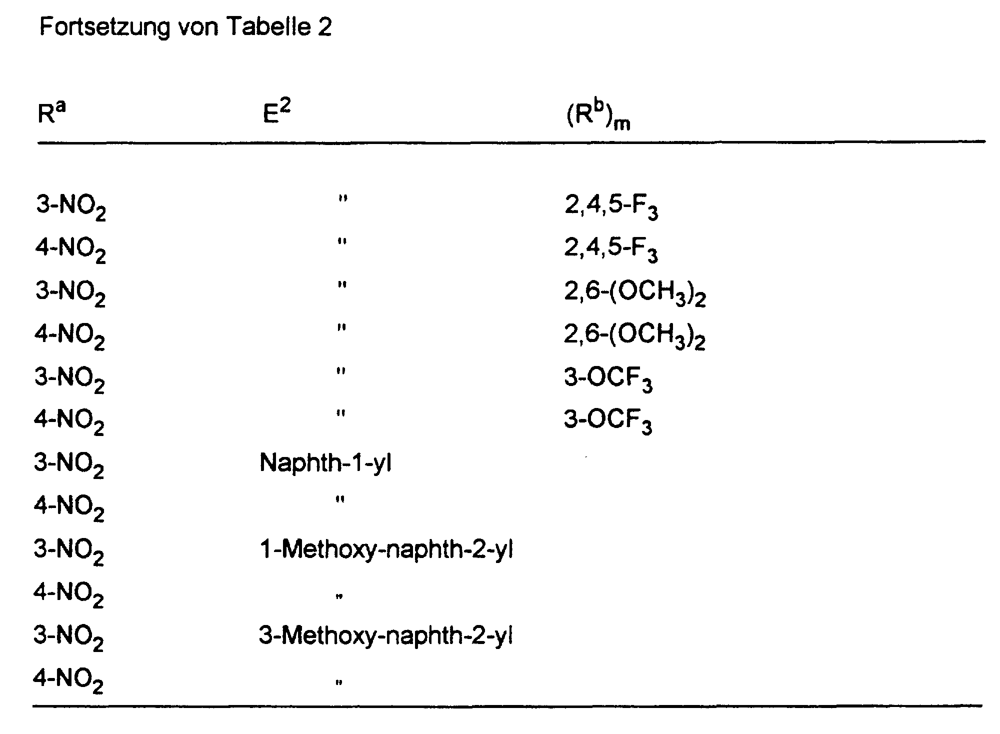

Die Herstellung der Verbindungen der Formel (IV) (Anbindung) gelangen in Ausbeuten von 85-95% d. Th. und Reinheiten von über 95%. Die Abspaltungen der angebundenen Acylsulfonamide durch Oxidation mit m-Chlorperbenzoesäure und β-Eliminierung analog der Methode in Beispielen 3.2 und 7 erfolgte ebenfalls in hohen Ausbeuten von über 90% d. Th. und Reinheiten von mehr als 95%.

### Beispiel 8

Entsprechend den obigen Beispielen 1 bis 7 und dem Schema 1 wurde ausgehend von der Verbindung N-(3-Methoxynaphthalin-2-ylcarbonyl)-4-nitrophenylsulfonamid der Formel (IIIa-N) [siehe Schema 2] nach Reaktion mit der Harz-Linker-Verbindung aus Beispiel 1b) analog zu Beispiel 2b) das Harz-Linker-Addukt (IVa-N) hergestellt. Die Reduktion der Nitrogruppe in Addukt (IVa) analog zu Beispiel 3.1 ergibt Verbindung (IVb-N), welche mit Carbonsäurechloriden aus Tabelle 3 analog Beispiel 3.2 zu entsprechenden Harz-Linker-Addukten der allgemeinen Formel (IVc-N) acyliert wird. Die anschließende Abspaltung vom Harz (vgl. ebenfalls Beispiel 3.2) ergibt die jeweiligen Sulfonamide der allgemeinen Formel (la-N) [siehe Schema 2 in Verbindung mit Tabelle 3].

Auf diese Weise wurden in einer Serie 45 Sulfonamide (la-N) in Reinheiten von 45 bis 100% und jeweils in einer Menge erhalten, die für Einzeltests auf biologische Eigenschaften (Gewächshausscreening) ausreichend war.

Die Strukturen der einzelnen Sulfonamide (la-N) wurden durch Vergleich (HPLC, DC) mit auf konventionellem Wege hergestellten Sulfonamiden oder durch übliche Methoden der Strukturaufklärung (z. B. Elementaranalyse, ¹H-NMR, IR, MS) bestätigt.

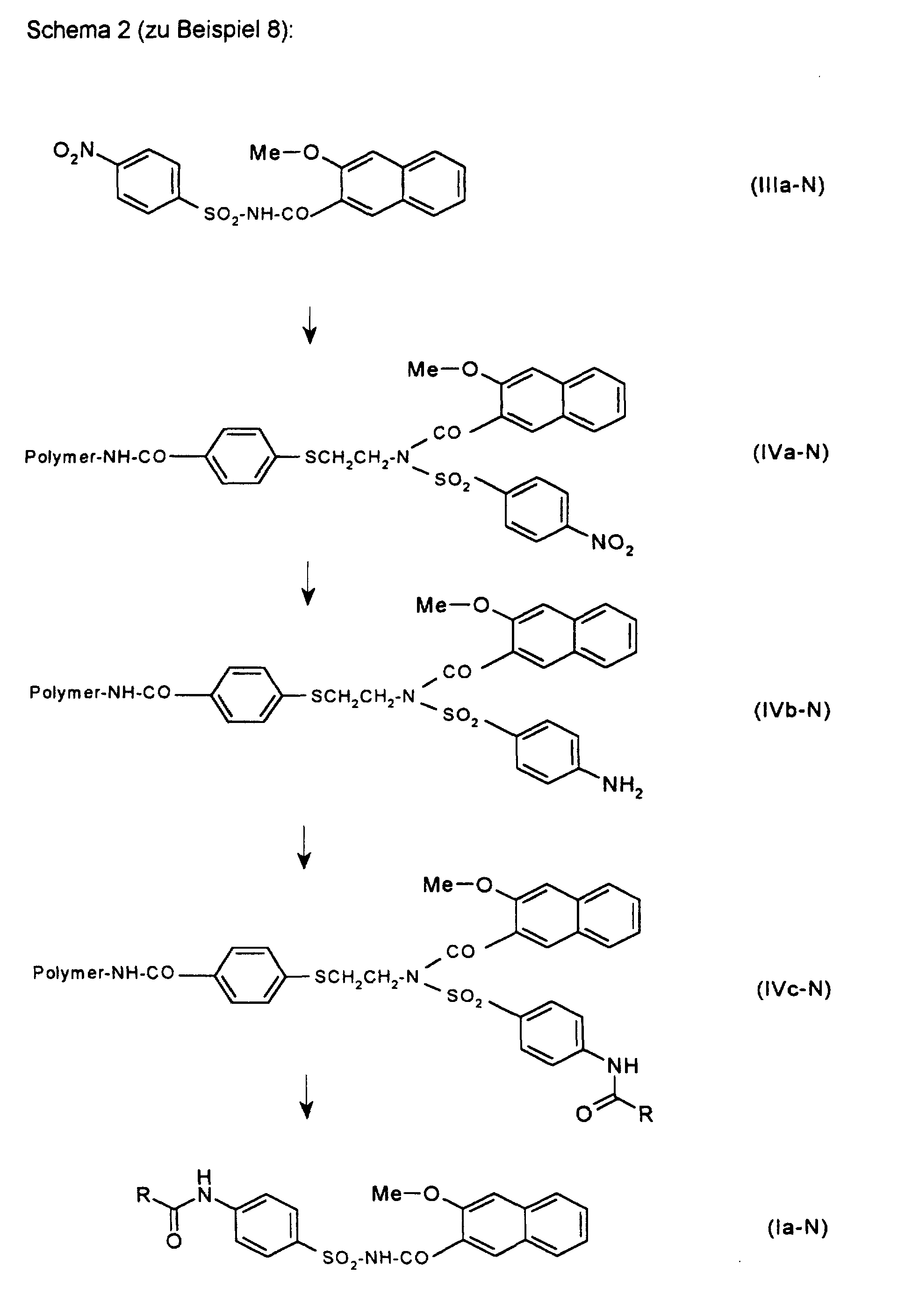

In Schema 2 und der nachfolgenden Tabelle 3 bedeutet "Me" jeweils Methyl.

**Tabelle 3:**

| Säurechloride der Formel (1) zur Umsetzung mit Harz-Linker-Addukt (IVb-N) R-CO-CI (1) | |
|---|---|
| Verb. Nr. | R |
| 1 | -CH(CH₃)CH₂CH₃ |
| 2 | Cyclohexyl |
| 3 | Adamant-1-yl |
| 4 | -C(CH₃)₂O-CO-CH₃ |
| 5 | -CH(C₆H₅)₂ |
| 6 | Thien-2-yl-methyl |
| 7 | Naphth-1-yl |
| 8 | 3,4-Dimethoxybenzyl |
| 9 | Pent-1-yl |
| 10 | -CH₂-O-CH₃ |
| 11 | Cycloprop-1-yl |
| 12 | Benzyl |
| 13 | n-Prop-1-yl |
| 14 | n-Hept-1-yl |
| 15 | -CH₂-O-C₆H₄-p-Me |
| 16 | -(CH₂)₁₆-CH₃ |
| 17 | Furan-2-yl |
| 18 | tert.-Butyl |
| 19 | -(CH₃)₁₅-CH₃ |
| 20 | ethyl |
| 21 | CH₂-O-CH₂-C₆H₅ |
| 22 | CH(C₂H₅)-O-C₆H₅ |
| 23 | CH(CH₃)-O-C₆H₅ |
| 24 | CH(C₂H₅)-n-C₄H₉ |
| 25 | CCl₃ |
| 26 | 3,4-Dichlorphenyl |
| 27 | 4-Trifluormethyl-phenyl |
| 28 | 2-Fluorphenyl |
| 29 | 4-Ethyl-phenyl |
| 30 | 4-Methyl-phenyl |
| 31 | 4-Fluor-phenyl |
| 32 | 2,4-Difluor-phenyl |
| 33 | 3-Trifluormethoxy-phenyl |
| 34 | 3-Chlor-phenyl |
| 35 | 2-Chlor-phenyl |
| 36 | 3,5-Dichlor-phenyl |
| 37 | 2-Brom-phenyl |
| 38 | 2,6-Difluor-phenyl |
| 39 | 2,6-Dichlor-phenyl |
| 40 | 2,4,5-Trifluor-phenyl |
| 41 | 4-Brom-phenyl |
| 42 | Phenyl |
| 43 | Thien-2-yl |
| 44 | Methyl |
| 45 | Isopropyl |

## Patentansprüche

1. Verfahren zur Herstellung von chemischen Verbindungen der Formel (I),
R¹-SO₂-NH-CO-R² (I)
worin R¹ und R² jeweils einen organischen Rest bedeuten,
**dadurch gekennzeichnet, daß** man
a) eine Harz-Linker-Verbindung der Formel (II),
[Harzpolymer]-[L-Nuc]ₙ (II)
worin
[Harzpolymer] für den Rest eines Harzes steht, der über n Bindungsstellen mit den dargestellten n Gruppen der Formel -L-Nuc verbunden ist,
L jeweils einen organischen Linker bedeutet,
Nuc eine nucleofuge Gruppe (Abgangsgruppe) oder unter den Reaktionsbedingungen zu einer Abgangsgruppe zu aktivierenden Gruppe bedeutet,
n die Anzahl der funktionellen Gruppen L-Nuc am Harz bedeutet und größer oder gleich 1 ist,
mit einem Acylsulfonamid der Formel (III),
E¹-SO₂-NH-CO-E² (II)
worin E¹ und E² unabhängig voneinander jeweils einen für die Herstellung der Reste R¹ bzw. R² in Verbindung (I) geeigneten organischen Rest bedeuten,
in Gegenwart eines Kondensationsmittels zu einem harzgebundenen Addukt der Formel (IV), 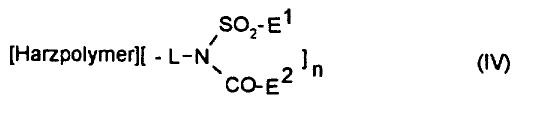 worin [Harzpolymer], L, n, E¹ und E² wie in Formel (II) bzw. Formel (III) definiert sind, umsetzt,
b) das erhaltene Addukt (IV) in einem oder mehreren weiteren Reaktionsschritten an den organischen Resten E¹ bzw. E² derivatisiert und damit gegebenenfalls über harzgebundene Zwischenprodukte der Formel (IV'), welche im Unterschied zu Formel (IV) die organischen Reste (E¹)' bzw. (E²)' der Derivate enthalten, zur Verbindung (IV"), 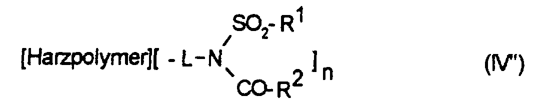 worin R¹ und R² wie in Formel (I) und [Harzpolymer], L und n wie in Formel (II) bzw. Formel (IV) definiert sind, umsetzt und
c) die Verbindung der Formel (I) aus dem Harz-Linker-Addukt der Formel (IV") abspaltet.

2. Verfahren nach Anspruch 1, **dadurch gekennzeichnet, daß** der Linker einer aus der Gruppe der Linker ist, die in der harzgebundenen Synthese für die Anbindung von Carbonsäuren eingesetzt werden können.

3. Verfahren nach Anspruch 1 oder 2, **dadurch gekennzeichnet, daß** in Formel (II) der Linker L-Nuc eine Gruppe der Formel -CO-p-C₆H₄-S-CH₂CH₂-OH bedeutet und die Verbindung (II) unter den kondensierenden Bedingungen, wie sie nach der Mitsunobu-Reaktion analog für Carbonsäuren angewendet werden, mit dem Acylsulfonamid als acider Komponente zum Harz-Linker-Addukt der Formel (IV) umgesetzt wird.

4. Verfahren nach Anspruch 3, **dadurch gekennzeichnet, daß** die Abspaltung der Verbindung der Formel (I) aus der Verbindung der Formel (IV") unter Oxidation des Schwefelatoms im Linker L zum Sulfon und anschließender β-Eliminierung erfolgt.

5. Verbindungen der Formel (IV), 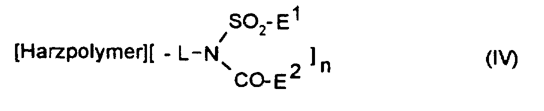 worin
[Harzpolymer] für den Rest eines Harzes steht, der über n Bindungsstellen und die Linker der Formel L mit den Gruppen der Formel E¹-SO₂-N-CO-E² verbunden ist,
L jeweils einen organischen Linker bedeutet,
n die Anzahl der über die Linker L gebundenen Gruppen am Harz bedeutet,
E¹, E² unabhängig voneinander jeweils einen organischen Rest bedeuten.

6. Verfahren zur Herstellung einer Verbindung der Formel (IV) nach Anspruch 5, **dadurch gekennzeichnet, daß** man eine Harz-Linker-Verbindung der Formel (II),
[Harzpolymer]-[L-Nuc]ₙ (II)
worin [Harzpolymer], L und n wie in Formel (IV) definiert sind und
Nuc eine nucleofuge Gruppe (Abgangsgruppe) oder unter den Reaktionsbedingungen zu einer Abgangsgruppe zu aktivierenden Gruppe bedeutet,
mit einem Acylsulfonamid der Formel (III),
E¹-SO₂-NH-CO-E² (III)
worin E¹ und E² wie in Formel (IV) definiert sind,
in Gegenwart eines Kondensationsmittels zu dem harzgebundenen Addukt der Formel (IV) umsetzt.

7. Verfahren nach Anspruch 6, **dadurch gekennzeichnet, daß** der Linker L-Nuc in Formel (II) eine Gruppe der Formel -CO-p-C₆H₄-S-CH₂CH₂-OH bedeutet und die Verbindung (II) unter den analogen Bedingungen für eine Mitsunobu-Reaktion mit dem Acylsulfonamid als acider Komponente zum Harz-Linker-Addukt der Formel (IV) umgesetzt wird.

8. Verfahren zur Herstellung von Verbindungen der Formel (IV"), 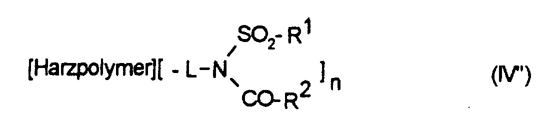 worin [Harzpolymer], L, n, R¹, R² wie in Formeln (I) und (II) in Anspruch 1 definiert sind, **dadurch gekennzeichnet, daß** man eine Verbindung der Formel (IV), 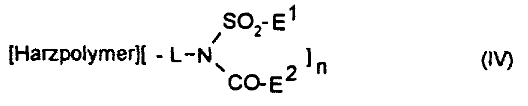 worin [Harzpolymer], L und n wie in Formel (IV'') definiert sind und
E¹, E² unabhängig voneinander jeweils einen für die Herstellung der Reste R¹ bzw. R² in Verbindung (IV'') geeigneten organischen Rest bedeuten,
in einem oder mehreren weiteren Reaktionsschritten an den organischen Resten E¹ bzw. E² derivatisiert und damit gegebenenfalls über harzgebundene Zwischenprodukte der Formel (IV'), welche im Unterschied zu Formel (IV) die organischen Reste (E¹)' bzw. (E²)' der Derivate enthalten, zur Verbindung (IV") umsetzt.

9. Verfahren zur Herstellung einer Verbindungen der Formel (I),
R¹-SO₂-NH-CO-R² (I)
worin R¹ und R² jeweils einen organischen Rest bedeuten,
**dadurch gekennzeichnet, daß** man die Verbindung der Formel (I) aus dem Harz-Linker-Addukt der Formel (IV"), 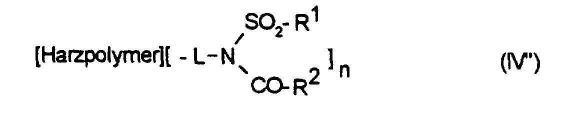 worin R¹ und R² wie in Formel (I) definiert ist und [Harzpolymer], L und n wie in Formel (II) bzw. Formel (IV") nach Anspruch 1 definiert sind, abspaltet.

10. Verfahren nach Anspruch 9, **dadurch gekennzeichnet, daß** der Linker L in Formel (IV") eine Gruppe der Formel -CO-p-C₆H₄-S-CH₂CH₂- bedeutet, wobei die Carbonylgruppe des Linkers am Harzpolymer gebunden ist, und die Abspaltung der Verbindung der Formel (I) aus der Verbindung der Formel (IV") unter Oxidation des Schwefelatoms im Linker L zum Sulfon und anschließender β-Eliminierung erfolgt.

## Claims

1. A process for the preparation of chemical compounds of the formula (I)
R¹-SO₂-NH-CO-R² (I)
in which R¹ and R² are each an organic radical, which comprises
a) reacting a resin-linker compound of the formula (II)
[resin polymer]-[L-Nuc]ₙ (II)
in which
[resin polymer] is the radical of a resin which is connected via n binding sites with the n groups of the formula -L-Nuc shown,
L is in each case an organic linker,
Nuc is a nucleofugic group (leaving group) or a group to be activated under the reaction conditions to give a leaving group,
n is the number of functional groups L-Nuc on the resin and is greater than or equal to 1,
with an acylsulfonamide of the formula (III)
E¹-SO₂-NH-CO-E² (III)
in which E¹ and E² independently of one another in each case are an organic radical which is suitable for the preparation of the radicals R¹ and R² in compound (I),
in the presence of a condensing agent to give a resin-bound adduct of the formula (IV) 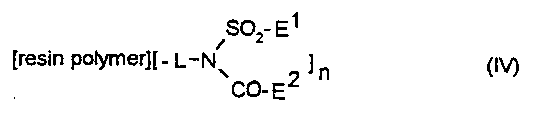 in which [resin polymer], L, n, E¹ and E² are as defined in formula (II) or formula (III),
b) derivatizing the adduct (IV) obtained in one or more further reaction steps on the organic radicals E¹ or E² and thus optionally reacting via resin-bound intermediates of the formula (IV'), which in contrast to formula (IV) contain the organic radicals (E¹)' or (E²)' of the derivatives, to give the compound (IV") 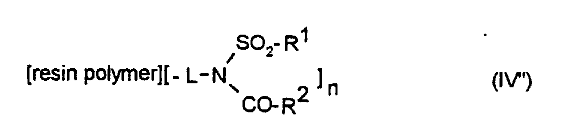 in which R¹ and R² are as defined in formula (I) and [resin polymer], L and n are as defined in formula (II) or formula (IV), and
c) cleaving the compound of the formula (I) from the resin-linker adduct of the formula (IV").

2. The process as claimed in claim 1, wherein the linker is a linker from the group of linkers which can be employed for the binding of carboxylic acids in resin-bound synthesis.

3. The process as claimed in claim 1 or 2, wherein in formula (II) the linker L-Nuc is a group of the formula -CO-p-C₆H₄-S-CH₂CH₂-OH and the compound (II) is reacted with the acylsulfonamide as acidic component to give the resin-linker adduct of the formula (IV) under the condensing conditions as are analogously used for carboxylic acids according to the Mitsunobu reaction.

4. The process as claimed in claim 3, wherein the cleavage of the compound of the formula (I) from the compound of the formula (IV") is carried out with oxidation of the sulfur atom in the linker L to the sulfone and subsequent β-elimination.

5. A compound of the formula (IV) 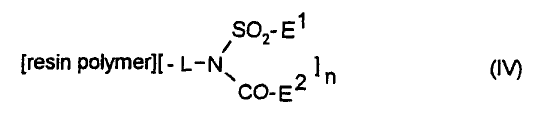 in which
[resin polymer] is the radical of a resin which is connected to the groups of the formula E¹-SO₂-N-CO-E² via n binding sites and the linkers of the formula L,
L is in each case an organic linker,
n is the number of the groups bonded to the resin via the linker L,
E¹, E² independently of one another in each case are an organic radical.

6. A process for the preparation of a compound of the formula (IV) as claimed in claim 5, which comprises reacting a resin-linker compound of the formula (II)
[resin polymer]-[L-Nuc]ₙ (II)
in which [resin polymer], L and n are as defined in formula (IV) and
Nuc is a nucleofugic group (leaving group) or group to be activated under the reaction conditions to give a leaving group,
with an acylsulfonamide of the formula (III)
E¹-SO₂-NH-CO-E² (III)
in which E¹ and E² are as defined in formula (IV), in the presence of a condensing agent to give the resin-bound adduct of the formula (IV).

7. A process as claimed in claim 6, wherein the linker L-Nuc in formula (II) is a group of the formula -CO-p-C₆H₄-S-CH₂CH₂-OH and the compound (II) is reacted with the acylsulfonamide as acidic component to give the resin-linker adduct of the formula (IV) under the analogous conditions for a Mitsunobu reaction.

8. A process for the preparation of compounds of the formula (IV") 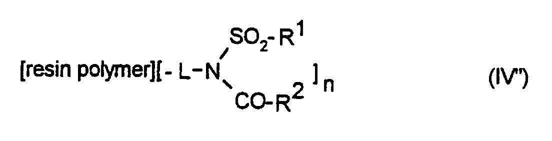 in which [resin polymer], L, n, R¹, R² are as defined in formulae (I) and (II) in claim 1, which comprises derivatizing a compound of the formula (IV) 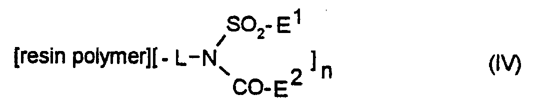 in which [resin polymer], L and n are as defined in formula (IV") and
E¹, E² independently of one another in each case are an organic radical suitable for the preparation of the radicals R¹ or R², respectively, in compound (IV"),
in one or more further reaction steps on the organic radicals E¹ or E² and thus optionally reacting via resin-bonded intermediates of the formula (IV'), which in contrast to formula (IV) contain the organic radicals (E¹)' or (E²)' of the derivatives, to give the compound (IV").

9. A process for the preparation of compounds of the formula (I)
R¹-SO₂-NH-CO-R² (I)
in which R¹ and R² in each case are an organic radical,
which comprises cleaving the compound of the formula (I) from the resin-linker adduct of the formula (IV") 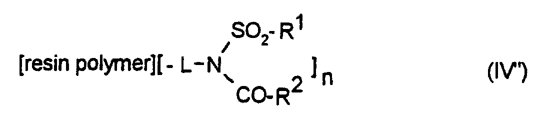 in which R¹ and R² are as defined in formula (I) and [resin polymer], L and n are as defined in formula (II) or formula (IV") as in claim 1.

10. The process as claimed in claim 9, wherein the linker L in formula (IV") is a group of the formula -CO-p-C₆H₄-S-CH₂CH₂-, the carbonyl group of the linker being bonded to the resin polymer, and the cleavage of the compound of the formula (I) from the compound of the formula (IV") taking place with oxidation of the sulfur atom in the linker L to the sulfone and subsequent β-elimination.

## Revendications

1. Procédé pour la préparation de composés chimiques de formule (I)
R¹-SO₂-NH-CO-R² (I)
où R¹ et R² représentent chacun un reste organique;
**caractérisé en ce qu'**on fait réagir
a) un composé résine-lieur de formule (II)
[polymère résineux]-[L-Nuc]ₙ (II)
où
[polymère résineux] L représente le reste d'une résine, qui est fixé par n sites de fixation à n groupes représentés de formule -L-Nuc, représente chacun un lieur organique
Nuc représente un groupe nucléofuge (groupe partant) ou un groupe à activer dans les conditions réactionnelles en un groupe partant,
n est le nombre de groupes fonctionnelles L-Nuc sur la résine et est supérieur ou égal à 1,
avec un acylsulfonamide de formule (III)
E¹-SO₂-NH-CO-E² (III)
où E¹ et E² représentent chacun, indépendamment l'un de l'autre, un reste organique approprié pour la préparation des restes R¹ ou R² dans le composé (I),
en présence d'un agent de condensation pour obtenir un adduit fixé à la résine de formule (IV) 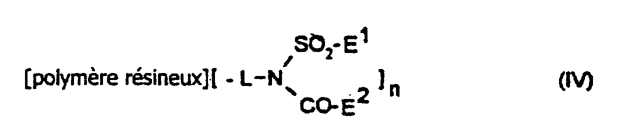 où [polymère résineux], L, n, E¹ et E² sont définis comme à la formule (II) ou formule (III),
b) on transforme l'adduit (IV) obtenu dans une ou plusieurs étapes réactionnelles ultérieures sur les restes organiques E¹ ou E² et par ce moyen on transforme éventuellement par l'intermédiaire des produits intermédiaires de formule (IV') fixés à la résine, qui contiennent à la différence de la formule (IV) les restes organiques (E¹)' respectivement (E²)' des dérivés, en le composé (IV"), 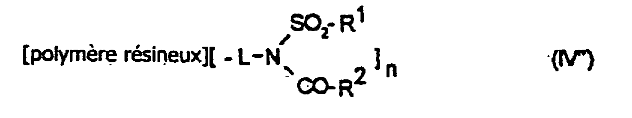 où R¹ et R² sont définis comme à la formule (I) et [polymère résineux], L et n sont définis comme à la formule (II) ou formule (IV), et
c) on sépare le composé de formule (I) de l'adduit résine-lieur de formule (IV").

2. Procédé selon la revendication 1, **caractérisé en ce que** le lieur est un lieur pris dans le groupe de lieurs que l'on peut utiliser dans la synthèse par fixation à la résine pour la fixation d'acides carboxyliques.

3. Procédé selon la revendication 1 ou 2, **caractérisé en ce que** à la formule (II) le lieur L-Nuc représente un groupe de formule -CO-p-C₆H₄-S-CH₂-CH₂-OH et on transforme le composé (II) sous les conditions de condensation qui sont utilisées selon la réaction de Mitsunobo de façon analogue pour les acides carboxyliques, par l'acylsulfonamide en tant que composant acide pour obtenir l'adduit résine-lieur de formule (IV).

4. Procédé selon la revendication 3, **caractérisé en ce que** la séparation du composé de formule (I) du composé de formule (IV") est effectuée sous oxydation de l'atome de soufre dans le lieur L en sulfone et par élimination β ultérieure.

5. Composés de formule (IV) 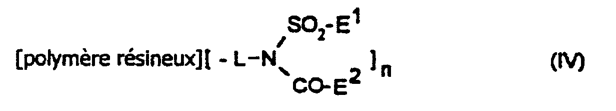 où
[polymère résineux] représente le reste d'une résine, qui est fixé par n sites de fixation aux groupes de formule E¹-SO₂-NH-CO-E²,
L représente chacun un lieur organique
n est le nombre de groupes fixés à la résine par le lieur L,
E¹, E² représentent chacun,
indépendamment l'un de l'autre, un reste organique.

6. Procédé pour la préparation d'un composé de formule (IV) selon la revendication 5, **caractérisé en ce qu'**on transforme un composé résine-lieur de formule (II)
[polymère résineux]-[L-Nuc] (II)
où [polymère résineux], L et n sont définis comme à la formule (IV) et
Nuc représente un groupe nucléophuge (groupe partant) ou un groupe à être activé en groupe partant dans les conditions réactionnelles,
par un sulfonamide de formule (III)
E¹-SO₂-NH-CO-E² (III)
où E¹ et E² sont définis comme à la formule (IV), en présence d'un agent de condensation pour obtenir un adduit fixé à la résine de formule (IV).

7. Procédé selon la revendication 6, **caractérisé en ce que** le lieur L-Nuc dans la formule (II) représente un groupe de formule -CO-p-C₆H₄-S-CH₂CH₂-OH et on transforme le composé (II) sous conditions analogues à une réaction de Mitsunobu par l'acylsulfonamide en tant que composant acide, pour obtenir l'adduit résine-lieur de formule (IV).

8. Procédé pour la préparation de composés de formule (IV") 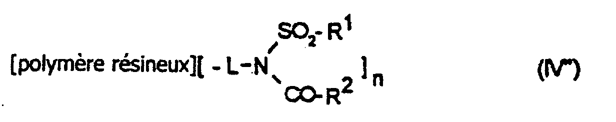 où [polymère résineux], L, n, R¹, R² sont définis comme aux formules (I) et (II) à la revendication 1, **caractérisé en ce qu'**on transforme un composé de formule (IV) 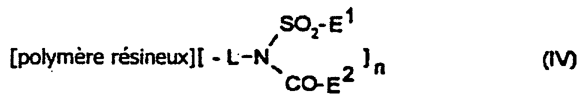 où [polymère résineux], L et n sont définis comme à la formule (IV") et
E¹, E² représentent chacun, indépendamment l'un de l'autre, un reste organique approprié pour la préparation des restes R¹ ou R² dans le composé (IV"),
dans une ou plusieurs étapes réactionnelles ultérieures sur les restes organiques E¹ ou E² et par ce moyen, on transforme éventuellement par l'intermédiaire de produits intermédiaires de formule (IV') fixés à la résine, qui présentent, à la différence de la formule (IV), les restes organiques (E¹)' ou (E²) ' des dérivés, pour obtenir un composé de formule (IV").

9. Procédé pour la préparation d'un composé de formule (I)
R¹-SO₂-NH-CO-R² (I)
où R¹ et R² représentent chacun un reste organique,
**caractérisé en ce qu'**on sépare
le composé de formule (I) de l'adduit résine-lieur de formule (IV') 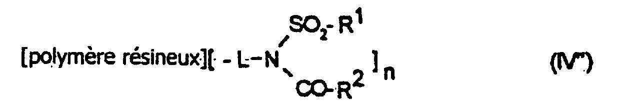 où R¹ et R² sont définis comme à la formule (I) et [polymère résineux], L et n sont définis comme à la formule (II) ou formule (IV") selon la revendication 1.

10. Procédé selon la revendication 9, **caractérisé en ce que** le lieur L à la formule (IV") représente un groupe -CO-p-C₆H₄-S-CH₂CH₂-, le groupe carbonyle du lieur étant fixé au polymère résineux, et on réalise la séparation du composé de formule (I) du composé de formule (IV") sous oxydation de l'atome de soufre L en sulfone et par élimination β ultérieure.
